Europäisches Patentamt

(19) European Patent Office

Office européen des brevets

(11) Publication number: **0 182 562 B1**

(12) **EUROPEAN PATENT SPECIFICATION**

(45) Date of publication of patent specification: **09.10.91** (51) Int. Cl.⁵: **C12N 15/75**, C12N 1/20, //C12R1/125

(21) Application number: **85308167.7**

(22) Date of filing: **08.11.85**

(54) **Process for a thermo-inducible gene expression in Gram positive bacteria, Bacillus subtilis strains containing plasmids which induce such an expression and portable vector systems.**

(30) Priority: **13.11.84 GB 8428594**

(43) Date of publication of application:
**28.05.86 Bulletin 86/22**

(45) Publication of the grant of the patent:
**09.10.91 Bulletin 91/41**

(84) Designated Contracting States:
**AT BE CH DE FR GB IT LI LU NL SE**

(56) References cited:
**EP-A- 0 130 074**
**US-A- 4 374 927**

**Biochem.Soc.Transactions 12 (June 1984), pp. 499-500**

**Journal of Virology, June 1980, vol. 34, no.3, pp. 789-791**

**Proc.Nat.Acad.Sci.USA 81 (Jan.1984), pp. 439-443**

(73) Proprietor: **SOLVAY & Cie (Société Anonyme)**
**Rue du Prince Albert, 33**
**B-1050 Bruxelles(BE)**

(72) Inventor: **Dhaese, Patrick, Dr.**
**Ertveldesteenweg 75**
**B-9969 Oostleeklo-Assenede(BE)**
Inventor: **Van Montagu, Marc, Dr.**
**Rue de Stassart 120**
**B-1050 Brussels(BE)**
Inventor: **Hussey, Caroline**
**Shelbourne Road 10**
**Dublin 4(IE)**

(74) Representative: **Smulders, Theodorus A.H.J., Ir. et al**
**Vereenigde Octrooibureaux Nieuwe Parklaan 107**
**NL-2587 BP 's-Gravenhage(NL)**

GENE, vol.16, nos.1-3, December 1981 (Amsterdam); J.J. SNINSKY et al.:"Construction and characterization of a novel two-plasmid system for accomplishing temperature-regulated, amplified expression of cloned adventitions genes in Escherichia coli", pp. 275-286

JOURNAL OF BACTERIOLOGY, vol. 113, no. 2, February 1973 (Baltimore, USA); R.E. YASBIN et al.:"Transformation and Transfection in Lysogenic Strains of Bacillus subtilis 168", pp. 540-548

## Description

The invention relates to a process for inducing gene expression in Gram positive bacteria by temperature variation. The invention provides also Gram positive bacteria of the Bacillus subtilis type containing at least two different kinds of plasmid capable of a temperature induced efficient expression of an heterologous structural gene cloned in one of those plasmids.

Up to now many authors have dealt in the literature with expression of heterologous structural genes in many kinds of prokaryotic and eukaryotic cells. The efficiency of the expression of such cloned heterologous structural genes is a function of many parameters of greater or lesser importance. Among those parameters the regulation of expression by an external modulator i.e. chemical or physical means allowing the choice of the exact moment at which the expression of the heterologous cloned structural gene is started, obviously is one of the most important factors. Many solutions for the resolution of this problem can be imagined, and one of these possible solutions consists in creating, one way or another, a temperature induced expression in order to start the expression of the heterologous structural gene as soon as a particular temperature is reached. The various ways in which this can be done are thoroughly discussed in publications such as US-A-4374927 patent of COHEN.

However such a regulated temperature induced gene expression has up to now been realised with satisfactory results only in bacteria of the Gram negative type such as Escherichia coli strains and has never been realised with Gram positive bacteria.

It has now been found that regulation of heterologous structural gene expression can under certain circumstances also be realised in Gram positive bacteria through a genetical construction as realised in the present invention.

Therefore one embodiment of the present invention relates to a process for a thermo-inducible gene expression in Gram positive bacteria characterized in that this thermo-inducible gene expression is realized through the introduction of two different kinds of plasmid, issued from genetic engineering and comprising respectively as concerns the first kind of plasmid the DNA material of a heterologous structural gene to be expressed preceeded by the DNA material of a promoter-operator appropriate to the Gram positive bacteria and as concerns the second kind of plasmid, the DNA material of a repressor gene, whose protein product is capable of interaction with the DNA material of the promoter-operator present on the first plasmid, and the DNA material of an entity temperature sensitive for replication of the second plasmid, into the Gram positive bacteria.

As the DNA material of the entity temperature sensitive for replication of the second plasmid, there can be used any known synthetic or naturally occurring DNA material which is insertable into the second kind of plasmid. Good results have been obtained by using as the second kind of plasmid, a plasmid which occurs in nature already as a temperature sensitive plasmid and wherein the DNA material of a repressor gene, whose protein product is capable of interaction with the promoter-operator of the first kind of plasmid, has been inserted by genetic engineering.

The promoter-operator and the repressor gene although located on two different plasmids should be able to interact with each other and are preferably issued from the same DNA material; as preferred DNA material the genomic material of a bacteriophage and more precisely of a temperate bacteriophage i.e. of the same temperate bacteriophage can be used.

As Gram positive bacteria there can be used such genera as Streptomyces, Corynebacterium, Clostridium, Staphylococcus and Bacillus. Good results have been obtained with Bacillus subtilis strains.

Consequently the invention relates also to new Bacillus subtilis strains containing two different kinds of plasmid issued from genetic engineering and comprising respectively as concerns the first kind of plasmid the DNA material of a heterologous structural gene to be expressed preceded by the DNA material of a promoter-operator appropriate to the Bacillus subtilis strain and as concerns the second kind of plasmid, the DNA material of a repressor gene, whose protein product is capable of interaction with the DNA material of the promotor present on the first plasmid, and the DNA material of an entity temperature sensitive for replication of the second plasmid.

The invention also comprises the new plasmids, the DNA material of the new promoter-operator and of the repressor gene as described hereafter. Moreover the invention relates also t a new plasmid-or vector composition intended to be introduced into Gram positive bacteria strains, and comprising two plasmids containing respectively:

(a) the DNA material of a heterologous structural gene from a procaryotic or eucaryotic cell preceeded by the DNA material of an early promoter-operator derived from a temperate phage for Bacillus subtilis, and

(b) the DNA material of a repressor gene, whose protein product is capable of interaction with the DNA material of the early promoter-operator present on the first plasmid, and the DNA material of an entity temperature sensitive for replication of the second plasmid.

The invention is illustrated hereafter in connection to a Bacillus subtilis strain and as a model for the heterologous structural gene there was chosen a chloramphenicol acetyl transferase (cat-86) gene. However, the invention can without any difficulties for the man skilled in the art be executed with other Gram positive bacteria and/or other heterologous structural genes originating from different procaryotic or eucaryotic cells. According to the particular nature of the heterologous structural gene used there can be synthetised different kinds of proteins.

Another embodiment of the present invention relates to portable bacterial control systems, for expression of a subject coding sequence, comprising a vector system containing a promotor/operator operably linked to the subject coding sequence and a DNA sequence encoding a compatible repressor protein of the operator wherein said operator and said DNA sequence encoding for the repressor protein are from Gram positive bacterial origin. Generally both operator and the DNA sequence encoding for the repressor protein are originating from the same Gram positive bacterial origin and the best results are obtained, up to now, when both are originating from the Bacillus subtilis temperate phage $\phi$105 and more particularly from the immunity region of this temperate phage.

The origin of the promotor is without direct importance for the invention as long as it remains compatible with the particular Gram positive bacterial host wherein expression is realised. It is thus possible to use a promotor originating from the same DNA material or from DNA material of another source as the DNA material from which the operator is originating. More particularly, promotors originating from Gram positive and Gram negative bacteria or even from eucaryotic origin can be used.

It is thus possible to chose, as a promotor for the present vector system, DNA material originating of various sources, having a very strong, a medium, a weak or even a very weak activity as can be desirable in particular cases.

Although various combinations of a repressor with its cognate operator are possible, the best results are up to now obtained with a repressor whose DNA material is from Gram positive bacterial origin and whose transcription is auto-regulated via its own promotor i.e. a repressor stimulating its own transcription.

The subject coding sequence which is intended to be expressed can be any DNA material, from procaryotic or eucaryotic origin, coding for one or more peptides or proteins of interest and valuable in technical fields such as pharmaceuticals for humans or animals, vaccines, enzymes and so on.

Any vector system can be used for this particular object of the present invention provided that it allows the incorporation of the above described essential elements. Good results have been obtained with a vector system based on plasmid technology. If such a plasmid technology is elected it remains however possible to incorporate the promotor/operator, the subject coding sequence and the DNA sequence encoding for the repressor protein on one and the same or on different plasmids and even, at least partially, on the chromosome of the host bacteria.

In the examples of the present invention there were used, to illustrate this object of the invention a system containing two kinds of plasmids and in order to induce, a thermo-inducible gene expression.

For the convenience of understanding the experimental section the following list of abbreviations and brief summary are given.

List of abbreviations

Ap = ampicillin; CAT = chloramphenicol acetyl transferase; Cm = chloramphenicol; EGTA = ethylene glycol-bis ($\beta$-aminoethyl ether) N,N,N,N'-tetraacetic acid; Em = erythromycin; EtBr = ethidium bromide; IPTG, isopropyl $\beta$-D-thiogalactopyranoside; kb = 1000 bp; Km = kanamycin; lacZpo = lac promoter operator region; LB = Luria broth; LBG = Luria broth containing glucose; MCS = multiple cloning site; pfu = plaque-forming units; R = resistance; S = sensitivity; Xgal = 5-bromo-4-chloro-3-indolyl $\beta$-D-galactopyranoside; [ ] = indicates plasmid carrier state.

Summary

A Bacillus subtilis/Escherichia coli plasmid vector containing a promoter-less chloramphenicol (Cm) acetyl transferase gene (cat-86) was constructed by ligation of pPL603 (reference 1) and pUC8 (Reference 2) at their unique EcoRI sites. Using this "promoter probe" vector we have obtained, by Cm resistance selection, a collection of cloned Sau3A fragments from the temperate phage $\phi$105 genome exhibiting promoter activity in Bacillus subtilis. Eighteen promoter plasmids were subsequently transferred to an acceptor cell BD170 [(pCGV28)] containing a functional $\phi$105 repressor gene inserted into the temperature-

EP 0 182 562 B1

sensitive replicon pE194. A repressor-controlled promoter was identified on the basis of its ability to confer thermo-inducible Cm resistance. The promoter is located on a 650 bp Sau3A fragment, mapping within the 3.2-kb EcoRI-F fragment, which also contains the φ105 repressor gene. By assaying cloned subfragments of EcoRI-F for expression of immunity against φ105 infection, the repressor gene could be assigned to a 1.1 kb EcoRI-HindIII fragment, which partially overlaps the promoter fragment.

EXPERIMENTAL WORK

1. Materials and methods

a) Bacterial strains, plasmids and enzymes

Bacterial hosts and plasmids from several sources are listed in Table I. Plasmids and strains constructed during this work are described in Tables II and III.

All cultures were grown in Luria broth containing 1% glucose (LBG), supplemented with the appropriate antibiotics. B. subtilis strains were maintained on M agar (Reference 3). For partial Sau3A digestion we used 0.2 units/μg DNA and 4 minutes incubation at 37°C.

b) DNA preparations

Small quantities of plasmid DNA were prepared from E. coli strains (Reference 4) and from B. subtilis by a modification of this method described by Goldfarb (reference 5). Larger plasmid preparations were made using scaled up versions of these methods, followed by CsCl-EtBr equilibrium gradient purification.

Phage φ105 was induced by the addition of mitomycin C (1μg/ml) to an exponentially growing culture (5 x 10^7 cfu/ml) of B. subtilis 168(φ105). After shaking for 3 hours at 37°C the lysed culture was cleared by centrifugation. The phage suspension was concentrated by polyethylene glycol precipitation (Reference 6) and resuspended in 1/100 vol. of phage buffer (10 mM Tris-HCl, pH 7.2, 10 mM MgSO₄). This suspension contained 10^11 pfu/ml.

After treatment with DNase (50μg/ml) and RNase (50μg/ml) for 1 hour at 37°C, the phage suspension was extracted twice with phenol and four times with ethyl ether. The DNA was precipitated with ethanol and recovered by spooling on a sterile glass rod.

c) Transformations

E. coli cells were transformed using the standard CaCl₂ technique (Reference 22). B. subtilis BD170 competent cells were prepared and stored as described by Dubnau and Davidoff-Abelson (Reference 7), and transformed in the presence of 1 mM EGTA (Reference 8). Selective antibiotic concentrations were : Km, 10μg/ml, and Cm, 15μg/ml, unless otherwise stated in the text. Cells transformed with pE194 or derivatives were induced for 1 h in the presence of Em (0.05μg/ml) prior to selection on plates containing 10μg/ml Em (Reference 9).

d) Assays for phage infectivity

Phage titres were determined as described by Rutberg (Reference 10), using exponentially growing cells of B. subtilis 168 as indicator bacteria.

Transformants were screened for sensitivity to φ105 by picking individual colonies into tubes containing 1 ml LBG. The tubes were incubated at 37°C until the cultures shoved a slight turbidity. Using a platinum loop, samples of each culture were streaked on LBG agar which had been impregnated with a dilute suspension of phage (5 x 10^3 pfu/plate). After incubation overnight at 33°C plaque formation was observed on sensitive strains. Strains that shoved immunity to infection with φ105 when tested in this way were also tested by mixing 0.3 ml of an exponentially growing culture with 0.1 ml phage suspension (3.5 x 10^3 pfu/ml). After incubation at 37°C for 15 min, 2.5 ml molten top-agar was added, and the mixture was poured on LB plates and incubated at 37°C. Exponentially growing cultures of strains 168 and 168(φ105) were used as sensitive and immune controls, respectively, in both tests.

2. Results

a) Construction of a B. subtilis/E. coli bifunctional promoter -probe vector

5

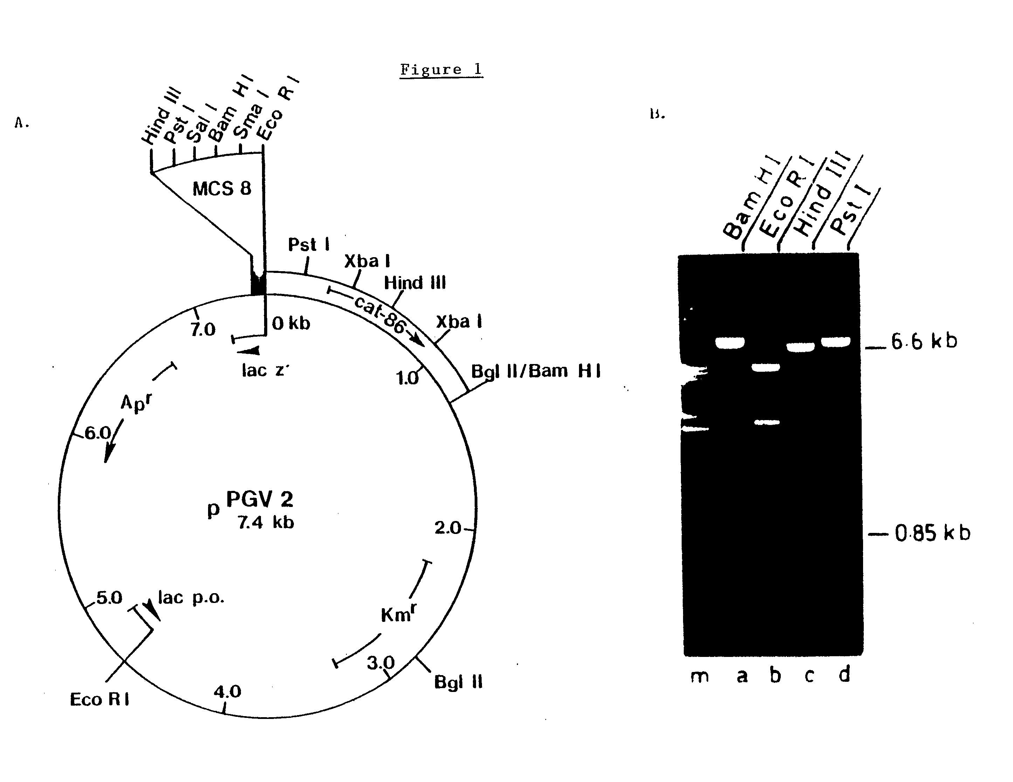

The 4.7-kb B. subtilis $Km^R$ plasmid pPL603 was derived from pUB110. It contains a silent $Cm^R$ gene (cat-86), originating from B. pumilus, that can be transcriptionally activated by cloning promoter sequences into the unique upstream EcoRI site. The sequence of the coding part of cat-86, and of its 5'-flanking region up to the EcoRI site, have been determined (References 11 and 12). Expression of cat-86 is Cm-inducible, most probably at the translational level (Reference 13). The EcoRI-linearized pPL603 was ligated to the EcoRI-cleaved E. coli replicon pUC8 $Ap^R$. After transformation of E. coli JM83 and selection for $Ap^R$ and $Km^R$, resistant colonies were checked for their white appearance on Xgal plates. The presence of a cointegrate plasmid in these colonies was further confirmed by agarose gel electrophoresis. Both types of recombinants, as defined by the two possible relative orientations of the composite replicons, were obtained. One type, designated pPGV2 (Fig. 1), was chosen for further study. This 7.4-kb plasmid replicates both in E. coli and in B. subtilis, but in the latter organism only the $Km^R$ gene is expressed. It is stably maintained in both species and no deletions were observed after long-term cultivation. B. subtilis cells harbouring pPGV2 show a reduced colony size on 5$\mu$g/ml Cm, and are sensitive to 10$\mu$g/ml. The cat-86 gene in pPGV2 is preceded by the pUC8-derived multiple cloning site sequence (MCS-8), containing unique SalI, SmaI, and BamHI sites. The latter site is located 358 bp upstream of the TTG initiation codon, and the intermediate sequence contains stop codons in all three reading frames.

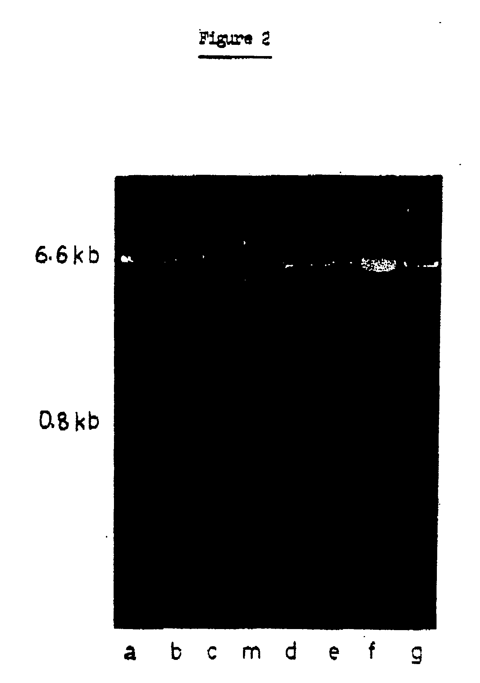

The usefulness of pPGV2 for cloning, by direct selection, of fragments exhibiting promoter activity in B. subtilis was investigated. To this end, chromosomal DNA from B. subtilis 168 was digested with Sau3A, ligated to BamHI-cleaved pPGV2, followed by transformation of B. subtilis BD170. Selection for $Cm^R$ (15$\mu$g Cm/ml) yielded resistant colonies at a frequency of 1% that of the $Km^R$ transformants. A convenient way to analyze the recombinant plasmid structure in these $Cm^R$ strains consisted of transforming E. coli C600r⁻m⁻ cells with the original B. subtilis plasmid preparation, followed by HindIII digestion of plasmid isolated from the E. coli transformants. As shown in Fig. 2 (lanes b and c), the increase in size of the small (0.85 kb) HindIII fragment indicates the length of the inserted promoter fragment. The level of cat-86 expression, as measured by the enzymatic assay described under Materials and Methods, varied considerably between strains carrying different recombinant plasmids, reflecting different promoter efficiencies. However, for all promoter plasmids examined CAT activity remained Cm-inducible.

### b) Cloning of promoters from the phage φ105 genome

Since no data were available concerning the location of transcriptional units in the DNA of phage φ105, it has been decided to construct a collection of φ105 promoter-containing plasmids representative of the entire genome. Purified φ105 DNA was digested with Sau3A under both complete and partial conditions (see Materials and Methods). The products of each mode of digestion were ligated to BamHI-cleaved pPGV2. After transformation of B. subtilis strain BD170, altogether more than 200 $Cm^R$ colonies were isolated. Of those, 35 were chosen for further study; their plasmids are listed in Table II. Strains harbouring pPGV1φ to pPGV10φ were randomly picked from the complete Sau3A transformants. Plasmids pPGV103φ to pPGV139φ are present in strains selected by replica plating of the complete Sau3A transformants on higher Cm concentrations (50-100-200$\mu$g/ml). Plasmids pPGV201φ to pPGV220φ were selected similarly from the partial Sau3A transformants. Table II shows that in all cases the $Cm^R$ phenotype is accompanied by the presence of an insert, the size of which ranges from 0.2-3.0 kb. The CAT activities directed by these φ105 promoter plasmids differ widely, ranging from a 10-fold (pPGV208φ) to a 3000-fold (pPGV138φ) increase over the activity measured for the vector pPGV2 (see also Table II).

Plasmid pPGV138φ confers at least a 10-fold higher CAT activity than pPL608, the latter being considered as a strong promoter plasmid, derived from pPL603 by the insertion of an EcoRI* fragment from temperate phage SP02 (Reference 14).

Sequence data on the pPGV138φ insert (not shown) indicate that it consists of a single, 386-bp Sau3A fragment containing a HindIII site (see also Fig. 2, lane e). A search for possible "-35" and "-10" regions within the sequence revealed a pair of correctly spaced hexanucleotides, 5'-TTGTTA-3' and 5'-GATAAT-3', showing homology with the canonical sequences proposed for a vegetative promoter recognized by$e$ $\sigma^{55}$-containing RNA polymerase (Reference 15). An AUG translational start codon is found 16 nucleotides downstream of the GATAAT, and is followed by an open reading frame running through the right border of the Sau3A fragment.

### c) Mapping of promoter fragments on the φ105 genome

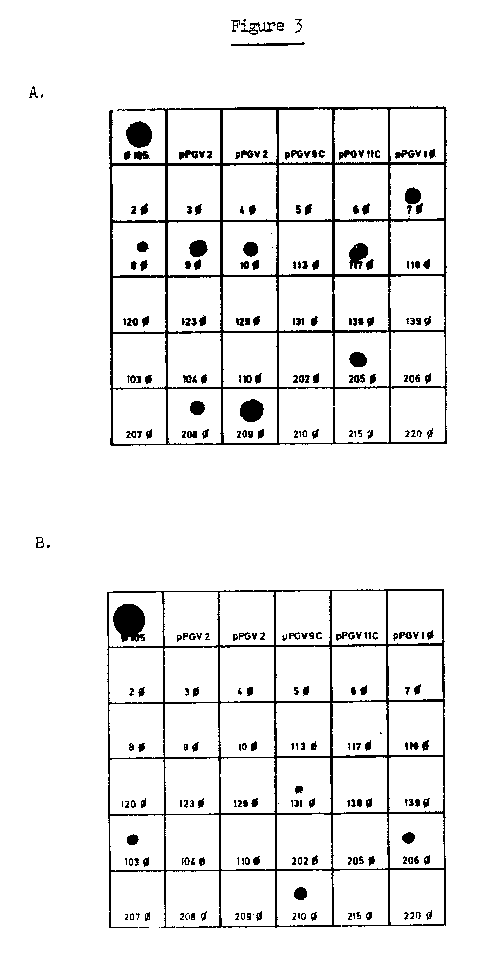

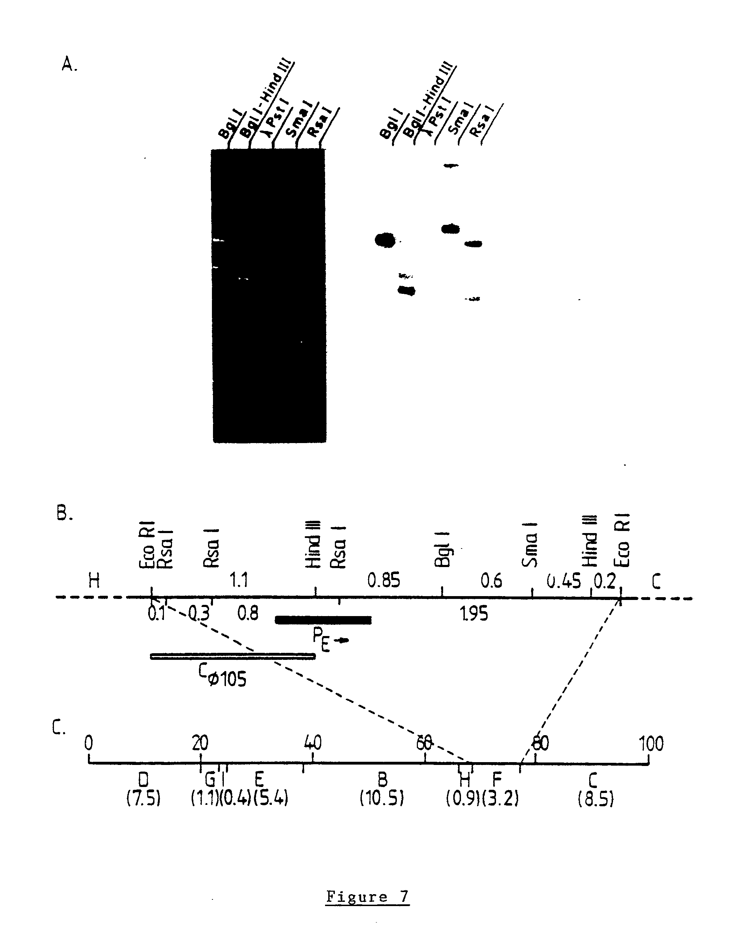

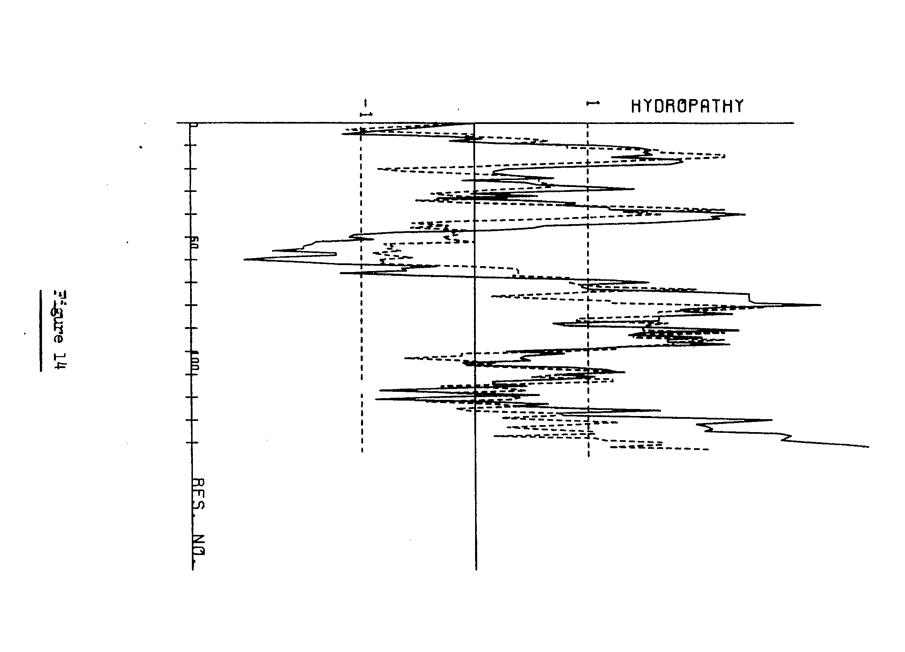

It is of interest to find repressor-controlled promoters amongst our collection of cloned φ105 fragments. A ³²P-labelled fragment as hybridization probe has used against the nitrocellulose-bound DNA of 31 different promoter plasmid isolates. As shown in Fig. 3A, 14 plasmids produced detectable hybridization signals, with considerable differences in their intensity. For example, strong hybridization was observed for pPGV209φ, whereas the signal obtained for pPGV131φ was very weak. The same filter was subsequently probed (Fig. 3B) with another φ105 fragment, the 5.4 kb EcoRI-E, which is not adjacent to EcoRI-F in the phage genome (see Fig. 7) but migrates nearest to the F fragment upon agarose gel electrophoresis of a φ105/EcoRI digest.

Comparison of Figs. 3A and 3B indicates that some plasmids (such as pPGV206φ, pPGV209φ and pPGV210φ) hybridize to both probes. This result cannot be explained solely by a slight degree of cross-contamination in the probes used, since other plasmids (such as pPGV7φ, pPGV10φ, pPGV103φ, pPGV205φ, and pPGV208φ) clearly hybridize specifically to either EcoRI-F or EcoRI-E. It is possible that some insert fragments are composed of two or more originally non contiguous Sau3A fragments. Taking further into account the size of the inserted fragment, the hybridization data can be summarized as follows (see also Table II) : plasmids pPGV7φ; pPGV8φ, pPGV9φ, pPGV10φ, pPGV117φ, pPGV205φ, pPGV208φ, and pPGV209φ contain φ105 sequences predominantly, if not entirely, originating from EcoRI-F; pPGV1φ, pPGV103φ, pPGV206φ, and pPGV210φ have inserts, the majority of which map in EcoRI-E.

### d) Cloning of the φ105 repressor gene in pE194

Clearly, a more conclusive screening for promoters subject to φ105 repressor-control should be based on their inability to promote cat-86 gene expression in a host cell containing functional repressor.

Therefore, the φ105 EcoRI-F fragment was inserted in a B. subtilis replicon that would be compatible with our promoter plasmids (which carry the replication origin of pUB110).

The $Em^R$ resistance plasmid pE194 (References 16 and 17) was chosen since it has an additional interesting property : it is naturally temperature-sensitive for replication (Reference 18). Consequently, it should be possible to eliminate the repressor gene at the nonpermissive temperature (45°C), thus mimicking the effect of a temperature-sensitive mutation in the repressor protein.

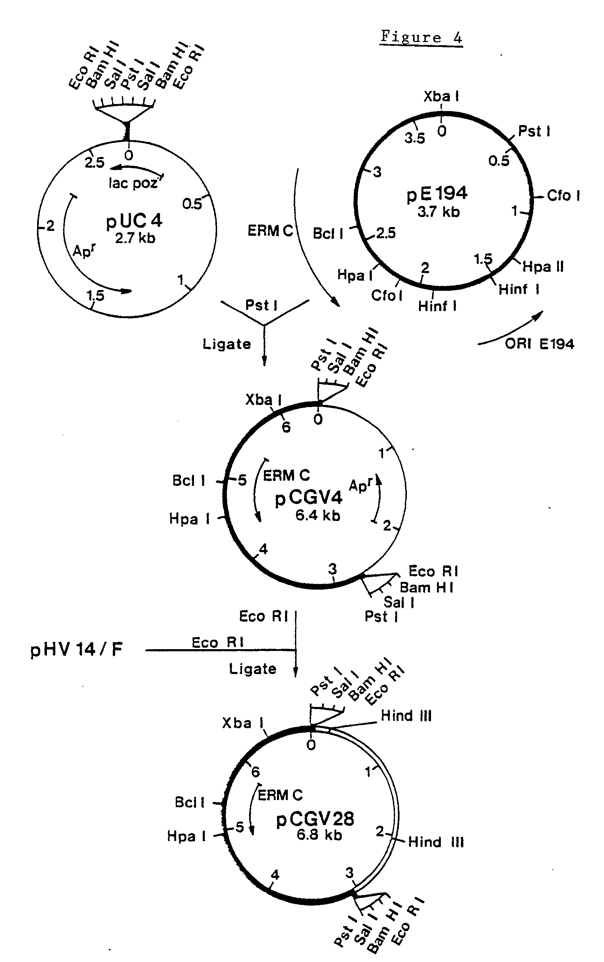

The low copy number of pE194 in B. subtilis and the paucity of unique restriction sites located outside the replication function or the $Em^R$ gene (Fig. 4) hampered its efficient use as a cloning vector. To overcome this problem, inserted PstI-cleaved pE194 DNA was inserted into the PstI site of the E. coli plasmid pUC4. The PstI site in pUC4 is part of the MCS7 element and is flanked symmetrically by two SalI, BamHI and EcoRI sites.

The resulting cointegrate plasmid pCGV4 replicates both in E. coli and B. subtilis, and serves as a convenient source of linear pE194 DNA with either PstI, SalI, BamHI or EcoRI ends.

To substitute the pUC4 moiety in pCGV4 by the EcoRI-F fragment of φ105, plasmid pHV14/F was digested with EcoRI and the products ligated to EcoRI-cleaved pCGV4. After screening 40 $Em^R$ B. subtilis BD170 transformants for immunity to φ105 infection at 37°C (see Materials and Methods), one immune colony was chosen for further analysis.

An EcoRI digest of the plasmid prepared from this strain showed two bands with sizes of 3.7 and 3.2 kb, corresponding to pE194 (as excised from pCGV4) and φ105-EcoRI-F, respectively. At 45°C the strain did not grow on Em plates, indicating that the pE194/EcoRI-F recombinant plasmid, designated pCGV28 (Fig. 4), has retained the temperature-sensitive character of pE194. This was further verified by overnight incubation of strains BD170 [pE194] and BD170 [pCGV28] in nonselective medium at 28°C, 33°C, 37°C, and 45°C followed by determination of the $Em^R$ fraction in the resulting cell populations. The results were similar for both strains : the cultures grown at 28°C contained essentially 100% $Em^R$ cells; at 33°C, 95%; at 37°C, 50%; and at 45°C, less than 0.1%.

### e) Identification of a negatively controlled φ105 promoter

To screen the collection of φ105 promoter-containing pPGV2 derivatives for the presence of repressor-controlled sequences, each of 18 different plasmids (see Table III) were individually introduced into competent cells of strain BD170 [pCGV28]. Transformants in each case were selected at 37°C on Em-Km plates, followed by testing of their Cm resistance (15μg/ml) at 33°C and 45°C. The expected phenotype for a strain in which cat gene expression would be under control of an early phage promoter is $Cm^s$ at 33°C, at which temper-ature a functional repressor is provided in trans, and $Cm^R$ at 45°C, at which temperature the repressor is eliminated due to loss of its carrier plasmid pCGV28.

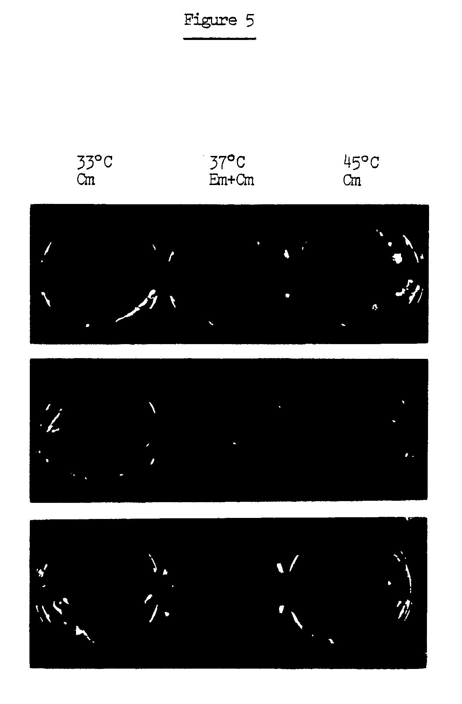

The results of such a test are summarized in Table III. Most pPGV plasmids conferred Cm resistance at both temperatures (see Fig. 5B), indicating that the cloned promoter was functionally repressor-independent. However, three plasmids, pPGV9φ, pPGV10φ and pPGV209φ, exhibited the expected temperatureinducible Cm$^R$ phenotype. As shown in Fig. 5 C, growth on Cm plates of strain BD170 [pCGV28,pPGV10φ] is marginal at 33°C, but normal at 45°C. The residual growth at 33°C can be explained by the partial loss of the repressor plasmid even at this temperature (see section d). In fact, the inhibiting effect of pCGV28 on cat gene expression directed by either pPGV9φ, pPGV10φ, or pPGV209φ is most strikingly seen when selective pressure is applied to maintain the repressor plasmid. Thus, strain BD170 [pCGV28,pPGV10φ] does not grow at all on Em-Cm plates at 37°C (Fig. 5). The same result was obtained for pPGV9φ and pPGV209φ. All other BD170 [pCGV28] transformants (see Table III) grew normally under these conditions.

Furthermore, several individual colonies of BD170 [pCGV28, pPGV10φ] isolated from a Cm plate at 37°C were tested for their Em resistance, and all were found to be sensitive. Conversely, all Em$^R$ colonies were Cm$^S$ (data not shown). Taken together, these results provide evidence for the repressor-controlled nature of the φ105 promoter sequences cloned in pPGV9φ, pPGV10φ and pPGV209φ. The approximate insert sizes for these three plasmids are 800, 650 and 1150 bp, respectively (see Table II).

f) Location of a φ105 early promoter fragment on the EcoRI-F restriction map

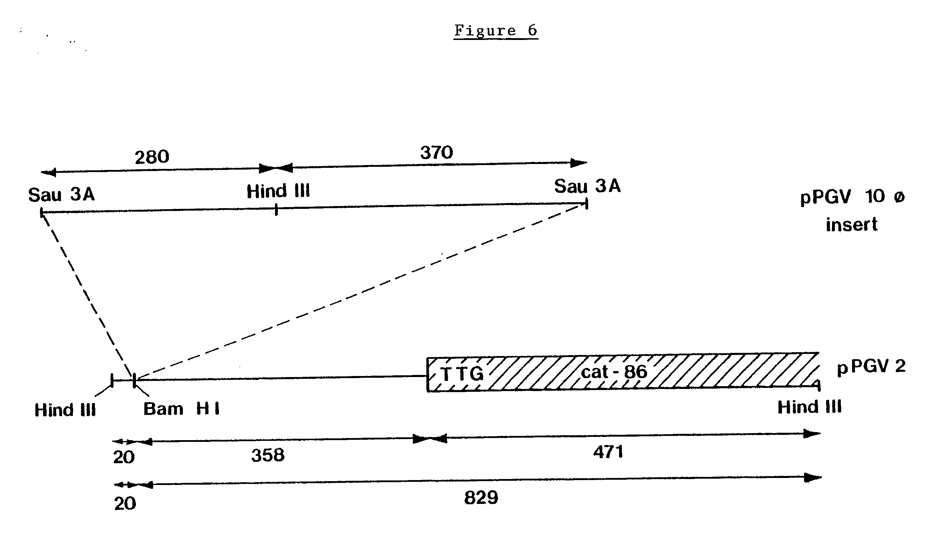

As shown above (Fig. 3A), the cloned φ105 Sau3A fragments on each of the three regulated expression plasmids hybridize to EcoRI-F. Fig. 2 (lane d) indicates that pPGV10φ contains an extra HindIII site compared to vector pPGV2, resulting in fragments of 1.2 and 0.3 kb. HindIII digestion of pPGV9φ generates an identical 1.2 kb fragment, and in addition a 0.45 kb fragment (data not shown). Thus, it is likely that both inserts share a common 650 bp Sau3A fragment with the HindIII site positioned approximately 370 bp from the cat-86 proximal insert border (Figure 6). Plasmid pPGV9φ most probably contains an additional 150 bp Sau3A fragment which is clearly not involved in the repressor-controlled cat-86 expression. The HindIII pattern of pPGV209φ (Figure 2, lane g) is more difficult to interpret : the insert contains two HindIII sites, giving rise to fragments of 1.3, 0.4 and 0.3 kb.

To construct a restriction map of φ105EcoRI-F, the fragment was cloned in pUC4 to generate the 5.9-kb pUC4/F hybrid plasmid, which was digested with several enzymes. The resulting map is shown in Fig. 7b. The orientation of fragment F with respect to the neighbouring phage sequences (EcoRI fragment H to the left, and fragment C to the right) was deduced from a BglI map of the complete φ105 DNA. Fig. 7A shows the results of hybridizing $^{32}$P-labelled pPGV10φ to the electrophoretically separated digestion products of purified EcoRI-F with the indicated enzymes. Homology was detected with the largest, 1950-bp EcoRI-BGlI fragment, and also with the largest EcoRI-SmaI fragment. Digestion with HindIII + BglI resulted in homologous fragments of 1100 and 850 bp, and RsaI produced hybridizing fragments of 2000 and 800 bp. From these results it is clear that the early promoter fragment overlaps the left HindIII site, 1.1 kb from the left border of EcoRI-F, and the RsaI site located 150 bp farther to the right. Digestion of the isolated 1.2-kb HindIII fragment of pPGV10φ with RsaI showed that the orientation of the promoter fragment is from left to right, such that it starts about 280 bp to the left of the HindIII site, and terminates about 230 bp past the RsaI site (Fig. 7B).

Probing of the same filter with $^{32}$P-labeled pPGV209φ produced an identical pattern of hybridizing fragments (data not shown). Hence, although pPGV209φ must contain additional sequences derived from elsewhere in the φ105 genome (as evidenced by the presence of a second HindIII site in the insert, which cannot possibly originate from EcoRI-F), the promoter region within this plasmid is probably identical to that of pPGV10φ.

These findings led us to map the φ105 repressor gene more precisely within EcoRI-F. Plasmid pUC4/F was digested with HindIII, and the two resulting fragments were cloned separately into HindIII-digested pPGV2 by substitution of the small 0.85-kb vector fragment. One type of recombinant contained the internal 1.9-kb HindIII subfragment of EcoRI-F, the other contained both EcoRI-HindIII external fragments (1.1 and 0.2 kb), with pUC4 inserted in-between. After transformation of B. subtilis BD170, φ105 infection assays were performed (see Materials and Methods, section..) on both types of transformants. These showed that cells containing the internal HindIII fragment remained sensitive to φ105, whereas the cloned external fragment combination conferred immunity to the host cell. Since the 0.2-kb EcoRI-HindIII fragment is probably too small to encode a functional repressor protein, we conclude that the φ105 repressor gene must reside within the left EcoRI-HindIII subfragment (Fig. 7B).

The following illustrates the nucleotide sequence and mutational analyses of the immunity repressor gene isolated from Bacillus subtilis temperate phage φ105.

g) The φ105 repressor is encoded by the promoter-proximal of two overlapping genes

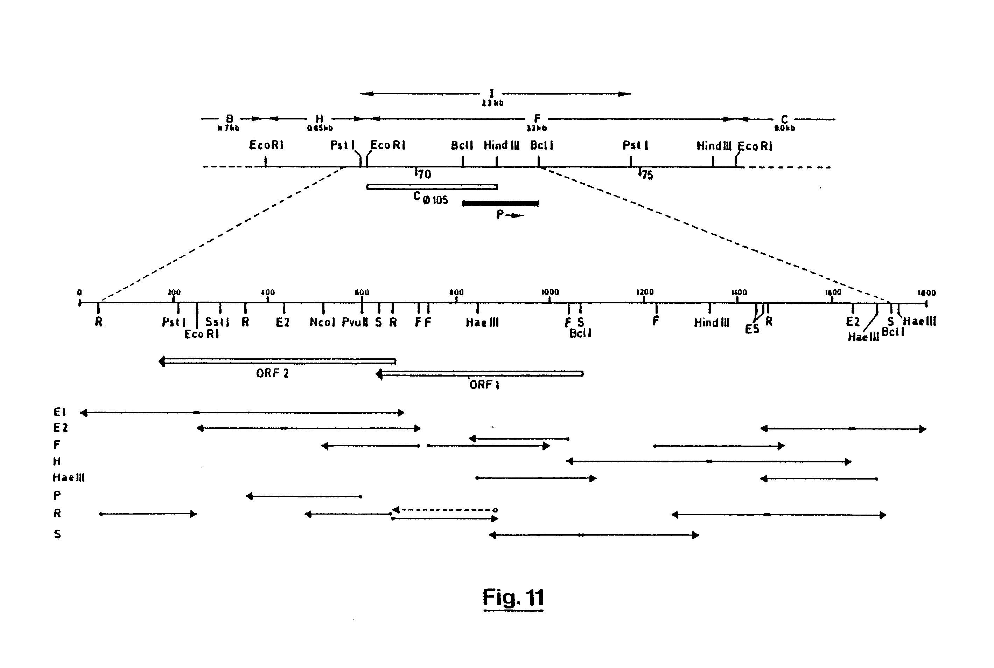

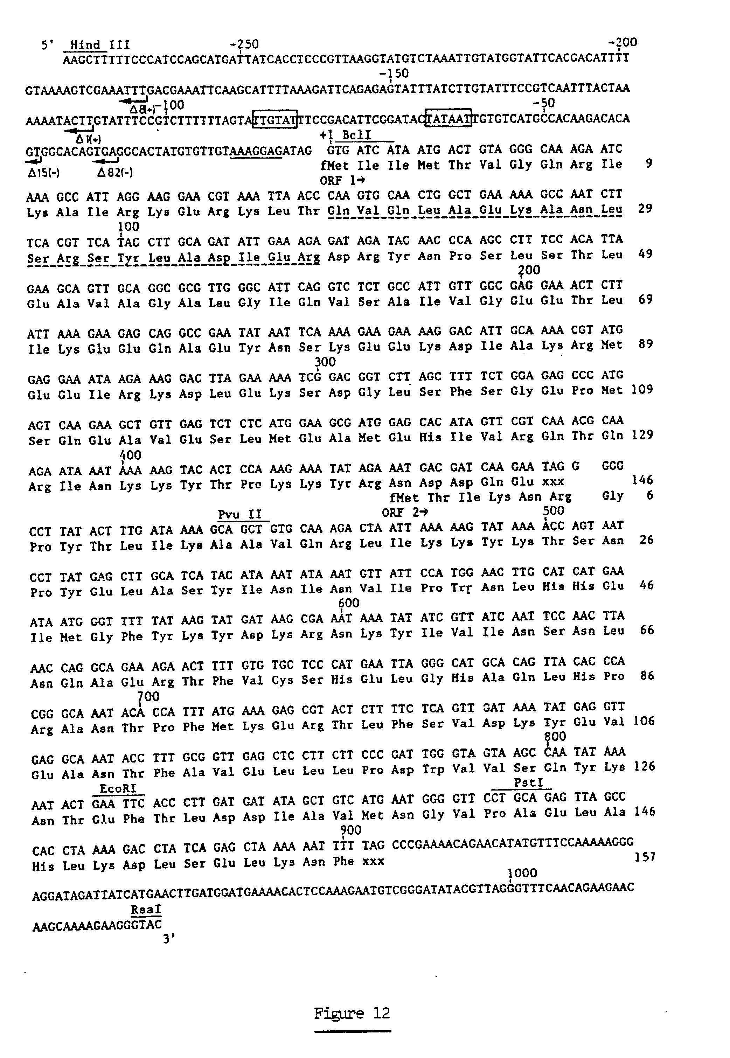

The left part of φ105 EcoRI fragment F, more specifically the 1100-bp EcoRI-HindIII subfragment (Figure 11), most probably encodes a repressor function by virtue of its ability to confer immunity to φ105 infection when inserted into a B. subtilis cloning vector. In order to determine the nucleotide sequence of this region of the phage genome, the 3.2-kb φ105 EcoRI-F fragment and the adjacent 0.9-kb EcoRI-H fragment were each cloned in the E. coli replicon pUC4, yielding respectively plasmids pUC4/F and pUC4/H. A detailed restriction map of the repressor region, together with the adopted Maxam-Gilbert sequencing strategy is shown in Figure 11. Both strands of a 1306-bp segment, extending from an RsaI site in EcoRI-H to the HindIII site, were completely sequenced. An additional sequence run, using plasmid pCGV14 DNA, from the leftmost RsaI site in EcoRI-F to the PstI site in EcoRI-H confirmed the contiguity of both EcoRI fragments. The sequence was analyzed for open-reading frames exceeding 300 nucleotides in length. Two partially overlapping open-reading frames were found, designated ORF1 and ORF2 in Figure 11. Both are orientated from right to left on the conventional φ105 map. The nucleotide and deduced amino acid sequences of the ORF1-ORF2 gene pair are shown in Figure 12. Since ORF2 encompasses the EcoRI site and is thus not entirely contained within the EcoRI-HindIII fragment, ORF1 at this stage seemed to be the most likely candidate to encode the repressor function. However, the possibility remained that the C-terminal part of the polypeptide is dispensable for repressor activity, and hence, ORF2 could not yet be definitely excluded.

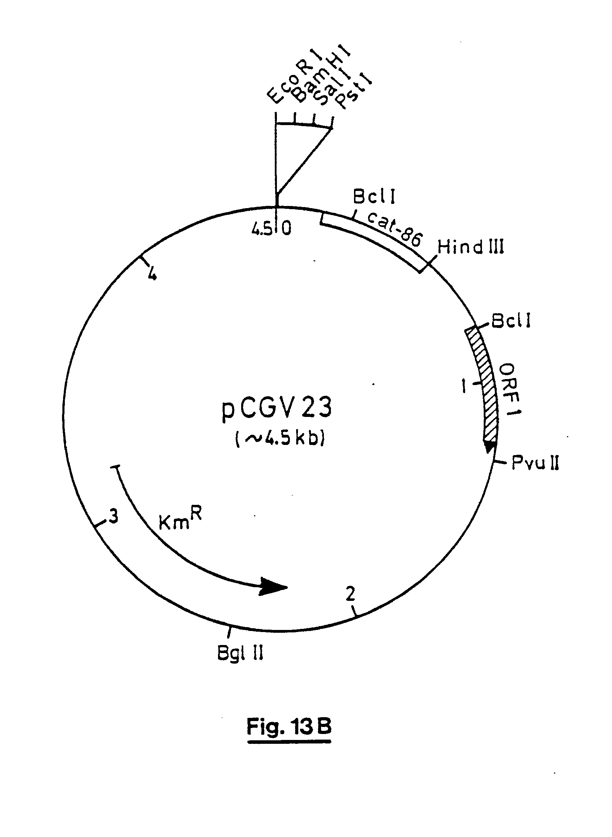

Figures 11 and 12 show the convenient location of a PvuII site with respect to the functional separation of both coding sequences : this site is well downstream from the stop codon or ORF1, whereas it occurs within the 14th codon from the N-terminus of ORF2. Hence, we decided to assay the 740-bp PvuII-HindIII fragment for repressor activity. The B. subtilis vector pPL703 containing unique HindIII and PvuII sites, was digested with both enzymes and ligated to HindIII + PvuII-digested pUC4/F. A large number of kanamycin-resistant B. subtilis transformants were subsequently tested for sensitivity to φ105. Only those cells containing a recombinant plasmid (designated pCGV23; see Fig. 13B) in which the 850-bp HindIII-PvuII vector fragment was substituted by the HindIII-PvuII fragment comprising ORF1, were found to be immune to φ105 superinfection. This finding conclusively demonstrated that ORF1 codes for the repressor.

Immediately preceding ORF1 is a sequence 5' AAAGGAG 3', showing a 7-bp complementarity to the 3' end of B. subtilis 16S rRNA, and hence representing a strong ribosomal binding site. In view of the frequent use of initiation codons other than ATG in B. subtilis, there are two possible candidates to serve as translational starts for the repressor : the GTG codon at position +1, and the in-frame ATG codon at position +10. At yet it has not been possible to discriminate between these two possibilities. The respective distances between these codons and the above-defined Shine-Dalgarno sequence are 4 and 13 bases, whereas on the average a spacing of 8 to 9 nucleotides is found for B. subtilis genes. Thus, the φ105 repressor monomer either consists of 146 or 143 amino acids, with calculated molecular weights of 16,745 or 16,387 daltons. These values correspond to a reasonable extent with the results obtained by examining the polypeptide synthesis pattern in B. subtilis minicells after infection with φ105. One of the earliest polypeptides, whose synthesis was not inhibited by chloramphenicol treatment, showed an apparent molecular weight of 18,000 on SDS-acrylamide gel electroforesis, and might therefore represent the product of this repressor gene.

Translation of ORF2 most probably starts at the ATG codon overlapping the triplets for residue 142 and 143 of the repressor. The function of the ORF2 gene product (157 amino acids) is unknown but its expression is obviously coregulated tightly with that of the repressor. This kind of translation overlap is quite unusual, but by no means unique.

h) Deletion Analysis of the 5' Upstream Control Region of the Repressor-ORF2 Genes

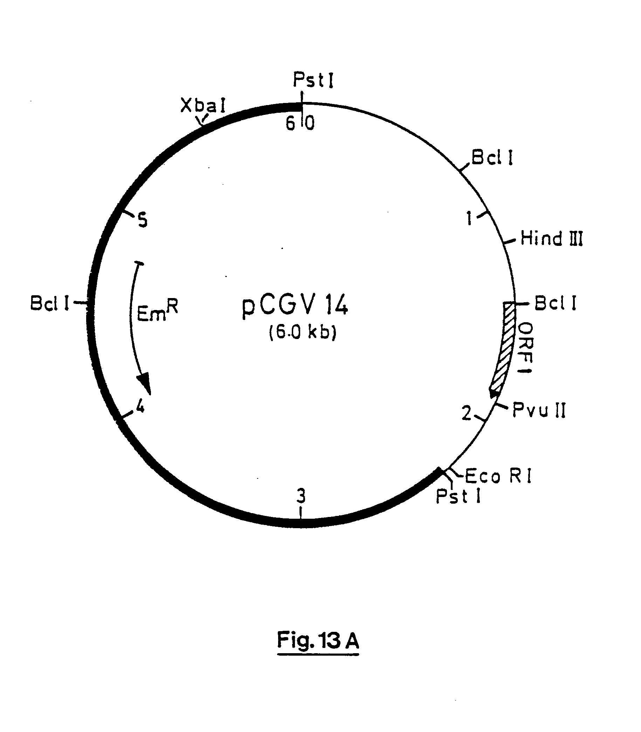

Having defined the structural part of the φ105 repressor gene, it is of interested to identify the control signals for the repressor-ORF2 transcription unit in the 5'-flanking DNA. Plasmid pCGV14 (Figure 13A) consists of the 2.3kb φ105 PstI-I fragment cloned in pE194 cop-6. It contains the intact ORF1 with over 1 kb of upstream sequence, and as expected it confers immunity to φ105 infection.

In pC6V14, the HindIII site 272 bp upstream of ORF1 is unique. To create a serie of deletions starting from this site and progressing towards the beginning of ORF1, HindIII-linearized pCGV14 DNA was treated with the Bal31 nuclease as follows : about 8μg of DNA in 0.1 ml buffer was digested with 1 unit of Bal31 at 30°C. Fractions were removed after 2.5, 5, 7.5, 12.5, and 15 minutes, and the extent of digestion was monitored by agarose gel electrophoresis. Subsequently, the appropriate fractions were pooled, phenol-extracted and ethanol-precipitated, followed by treatment with the Klenow fragment of DNA polymerase I. The resulting blunt-end fragments were self-ligated using T4 DNA ligase in the presence of 0.5 mM spermidine, prior to transformation of B. subtilis BR151. Individual erythromycin-resistant transformants were tested for phage φ105 infectivity, and their plasmid content was analyzed using BCII and PstI

digestions to approximately determine the extent of the deletion. Subsequently, the deletion endpoints in a few of the pCGV14Δ plasmids were determined at the nucleotide level.

As a result there were obtained a collection of pCGV14 mutants (pCGV14Δ...) with deletions extending in both directions from the HindIII site, and ranging in size from less than 100 to over 2000 bp. In this collection the two phenotypic classes were represented : some pCGV14Δ plasmids had retained the superinfection immunity phenotype of the parent pCGV14 whereas in the majority it was lost. All plasmids with a deletion extending beyond the BclI site at the N-terminus of ORF1 fell into the latter category, indicating that an intact ORF1 is not only sufficient, but also necessary for superinfection immunity. Moreover, some deletion plasmids, such as pCGV14Δ15 and pCGV14Δ82, still contained this BclI site and yet gave rise to φ105-sensitive cells. Sequence analysis (Figure 12) showed that pCGV14Δ15 has retained 36 nucleotides upstream of the GTG, and pCGV14Δ82 ends at -26. The deletion endpoints in two immunity-conferring plasmids, pCGV14Δ1 and pCGV14Δ8, were localized at -109 and -182 respectively. These results clearly indicate that the region between -109 and -36 upstream from the GTG is required for the expression of superinfection immunity, even from a multicopy plasmid. It is probable that the promoter for the repressor-ORF2 transcription unit is contained within this region. Figure 12 shows the presence of two hexanucleotides, 5' TTGTAT 3' and 5' TATAAT 3' separated by exactly 17 bp. In view of the homology between these sequences and the consensus for a$\sigma^{55}$- dependent promoter, it is likely that these serve as "-35" and "-10" recognition sites for the major vegetative form of B. subtilis RNA polymerase. This is consistent with the idea that the repressor is probably one of the first phage genes to be expressed after infection, and therefore its transcription should rely entirely upon the host machinery.

It is further noted that these promoter elements are preceded by a very AT-rich region, (76 % between positions -150 and -90), a feature common to several other B. subtilis genes.

### i) The φ105 repressor polypeptide : properties and comparison with known repressors

The codon usage and amino acid compositions for φ105 repressor and ORF2, as deduced from the nucleotide sequence, are shown in Table V.

In what follows, there was tentatively assigned translation initiation to the GTG codon and assumed that the terminal residue of the mature repressor polypeptide is isoleucine (Figure 12). The repressor does not contain cysteine or tryptophan, but has a high content of glutamic acid (20 residues). Its pH is likely to be close to neutral, given the sum of acidic residues (Asp + Glu = 27), and basic residues (Arg + His + Lys = 26). The most striking overall property of the polypeptide is its pronouced hydrophilicity, as illustrated in the hydropathy profile (Figure 14). Very hydrophilic regions extend from residues 10-19, 36-41, 71-100 and 130-146, whereas the only hydrophobic regions are found between residues 1-5 and 51-64. Clearly, this feature refers to the cytoplasmic localization of the repressor protein.

In order to elucidate which region(s) of the polypeptide might be involved in DNA binding, an extensive comparison of its amino acid sequence with that of several other repressors functional in Gram-negative bacteria such as E. coli or S. typhimurium was carried out. These included : λcl repressor, λcro protein, phage P22 c2 repressor, lac repressor, gal repressor, and the lexA gene product. The most favorable alignment for each pair of sequences was obtained using the Needleman-Wunsh algorithm, as modified by Kanehisa.

Table VI summarizes the results for each pairwise comparison. The overall homology, at the primary structure level, between φ105 and any of the other repressors is insignificant as the highest proportion of identical residues, obtained with the lexA protein, amounts to less than 18 %. However, figure 5 shows that there may exist some meaningful local homology in the N-terminal regions of φ105 repressor and phage p22 c2 repressor. On a total of 57 compared positions, 17 homologies are found without the need to introduce gaps. This could be relevant in new of the fact that phage φ105 contains two immunity regions. It is probable that this sequenced gene corresponds to the immC region of phage P22, and that the other φ105 region (located in the EcoRI-B fragment) is analogous to the mnt/ant (or immI) region of P22.

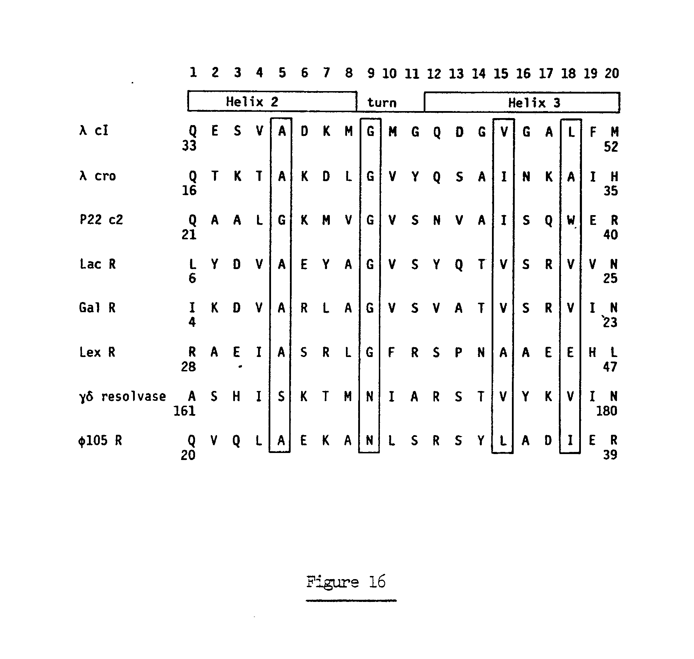

A further comparison step was based on the well documented observation that all known repressors, regardless of the specific operator sequence to which they bind, contain a stretch of 20 contigous residues exhibiting an α-helix-turn-α- helix configuration. This three-dimensional structural element is essential for interaction with the major groove of the DNA helix. The φ105 sequence was examined for regions whose sequence would be compatible, at each crucial position, with the sequences of this DNA-binding domain in a number of other repressors. As a result the region extending from position 20 (Gln) to 39 (Arg) is the most likely candidate. In Figure 16 this region was aligned with that of seven other repressors. The most important positions are 5, 9, 15 and 18 which should have alanine, glycine, and two hydrophobic amino acids, respectively. The φ105 sequence conforms well in three of these positions, whereas in position 9 it has asparagine which is, however, also found at that position in $\gamma\delta$-resolvase. Furthermore, hydrophobic residues should be present in positions 4, 8 and 19. The examined $\phi$105 sequence again agrees at two of these positions (4-Leu and 8-Ala), and the glutamic acid at 19 is also present in the P22 c2 repressor. Lastly, hydrophilic residues should occupy positions 1-3, 6-7, 11-14 and 16-17. Again, the $\phi$105 sequence shows hydrophilic side chains in 9 out of these 11 positions.

It should be noted however, that a $\phi$105 promoter-operator site controlled by the repressor has been localized within the 662-bp BclI fragment (Figure 11) immediately upstream from the repressor coding sequence (7). This promoter directs transcription from left to right, and therefore can be topologically considered the equivalent of $\lambda p_R$ with respect to the $\phi$105 repressor. In addition, this promoter has been inserted in front of the cat-86 gene in plasmid pPGV10$\phi$. Using such a hybrid construction, it is possible to assess the repressor function more directly, by testing the chloramphenicol resistance of B. subtilis - (pPGV10$\phi$) strains transformed with the different repressor plasmids constructed during this work, such as pCGV14 and its deletion derivatives. A perfect correlation was found between the superinfection immunity phenotype and the inhibition of cat-86 expression, resulting in Cm sensitivity of the double transformant. A $\phi$105-sensitive phenotype, on the other hand, was always accompanied by the restoration of cat-86 expression, giving rise to $Em^R Cm^{Rh}$ cells.

There are, however, obvious differences in the organization cf the $\phi$105 immunity region compared to that of $\lambda$ or the immC region of phage P22. First, the $\phi$105 repressor, unlike its Gram-negative counterparts, forms part of a translationally overlapping pair of cistrons. The function of ORF2 as well as the reason why its expression should be coordinated so tightly with that of the repressor are intriguing questions for which there is no answer to date.

Second, preliminary mapping data on the above mentioned target promoter-operator site indicate that it is located to the right of the HindIII site (Figure 11), and hence, at least 272 bp removed from the repressor initiation codon. In $\lambda$ and P22, the $p_R$-$O_R$ signals are immediately adjacent to the repressor coding sequence.

These results suggest further that the $\phi$105 repressor transcript, starting most likely some 10 bp downstream of the "TATAAT" hexanucleotide in Figure 12, contains at least 50 bases of untranslated leader, including a strong ribosomal binding site. Again, this is different from $\lambda$ cI whose transcription from the $p_{RM}$ promoter, initiates at the ATG codon and the P22 c2 maintenance RNA which contains only four 5'-untranslated nucleotides.

Using a comparative approach, there was identified a DNA-binding region within the $\phi$105 repressor polypeptide, extending from residue positions 20 to 39. It is now possible to test this assignment by targeted mutagenesis of the corresponding DNA sequence.

j) The $\phi$105 repressor stimulates its own transcription

One important aspect of the expression systems based on $\lambda P_L/P_R$ repressor elements, is the fact that $\lambda$ repressor binds to its own promoter (the $P_{RM}$ or "maintenance" promoter) and thereby stimulates transcription from this promoter. It was therefore of interest to examine whether the $\phi$105 repressor interacts with its promoter in a similar way. Figures 11 and 12 show that the central BclI fragment in fact contains two promoters : (i) the early promoter, which is negatively controlled by the repressor, and orientated from left to right on the $\phi$105 map, and (ii) at the other end, the ("maintenance") promoter for the repressor itself, orientated from right to left. In view of the spatial separation of these two promotors it was not evident that the same mechanism should apply.

In plasmid pPGV10$\phi$ (see above) this BclI (or Sau3A) fragment had been inserted upstream of the cat-86 gene in the left to right orientation, such that cat-86 expression is controlled by the early promoter.

To test whether the "maintenance" promoter is capable of transcriptionally activating the cat-86 gene, the BclI fragment was inserted in the reverse orientation, thus positioning the "maintenance" promoter immediately upstream of the cat-86 gene.

The resulting plasmid, pPGV17$\phi$, did not confer chloramphenicol resistance (15$\mu$g/ml), indicating that the $\phi$105 "maintenance" promoter, when dissected from its coding sequence, is not active. However, when active repressor was introduced in the same cell, by transformation with plasmid pCGV14, the resulting double transformant BR151(pPGV17$\phi$, pCGV14) showed an $Em^R Cm^R$ phenotype. This clearly indicates that repressor protein is required to activate the promoter for its own gene.

k) Construction of a hybrid promoter-operator for high-level, regulated gene expression in Bacillus subtilis

1) Construction of pPGV301

Having defined the two essential components of our thermo-inducible gene expression system, i.e. the repressor and operator fragment of $\phi$105, we sought to improve the efficiency of the system. Indeed, due to the weak promoter activity associated with the 650-bp Sau3A fragment of pPGV10$\phi$, the expression level of any foreign gene in the induced state (i.e. in the absence of repressor) would be rather low, and consequently the system would be prac tically of limited usefulness. The system could be considerably improved by combining the activity of a different, but very strong promoter with the regulatory properties of the operator site present in the 650-bp Sau3A fragment of pPGV10$\phi$.

In fact, the collection of promoter plasmids already contained such a strong, promoter : it is present on plasmid pPGV138$\phi$(see description p. 10) which confers at least a 100-fold higher CAT activity than pOGV10$\phi$(Table II). Consequently, plasmid pPGV138$\phi$ was used as starting material for the construction of a hybrid promoter-operator. To this end, the operator site of pPCV10$\phi$ had to be isolated and inserted between the strong promoter and the cat-86 test gene in pPGV138$\phi$. A unique Smal site was available, allowing the insertion of any blunt-end fragment.

As a first approach to defining more precisely the position of the operator region within the 650-bp Sau3A fragment, the effect of deleting the 300-bp HindIII fragment (fig. 6) from pPGV10$\phi$ was examined. The resulting deletion mutant, pPGV10$\phi$Δ8, retained all properties of the parent plasmid, indicating that the essential regulating elements were located within the right 370-bp HindIII-Sau3A part. This region was sequenced (fig. 8) and a 231-bp Rsal-Haelll fragment was subsequently chosen for insertion in the Smal site of pPGV138$\phi$, in both possible orientations. This yielded two new derivate plasmids, pPGV301 (with the operator in the correct orientation; see Fig. 9) and pPGV326 (with the inverse orientation). These plasmids, together with pPGV138$\phi$, were introduced into B. subtilis cells with or without a functional repressor.

## 2) cat-86 expression assays

To assess whether the hybrid promotor-operator element can direct high-level, repressor-regulated expression of the test gene, cat-86, the chloramphenicol resistance level (on petri dishes) of B. subtilis strains carrying pPGV301, in the absence and presence of the repressor was examined. As controls, PPGV138$\phi$ (without operator) and pPGV326 (with the operator in the wrong direction) are used.

As source of functional repressor, the Em$^R$ plasmid pCGV14 was used. The repressor is maintained by selection for Erytromycin resistance.

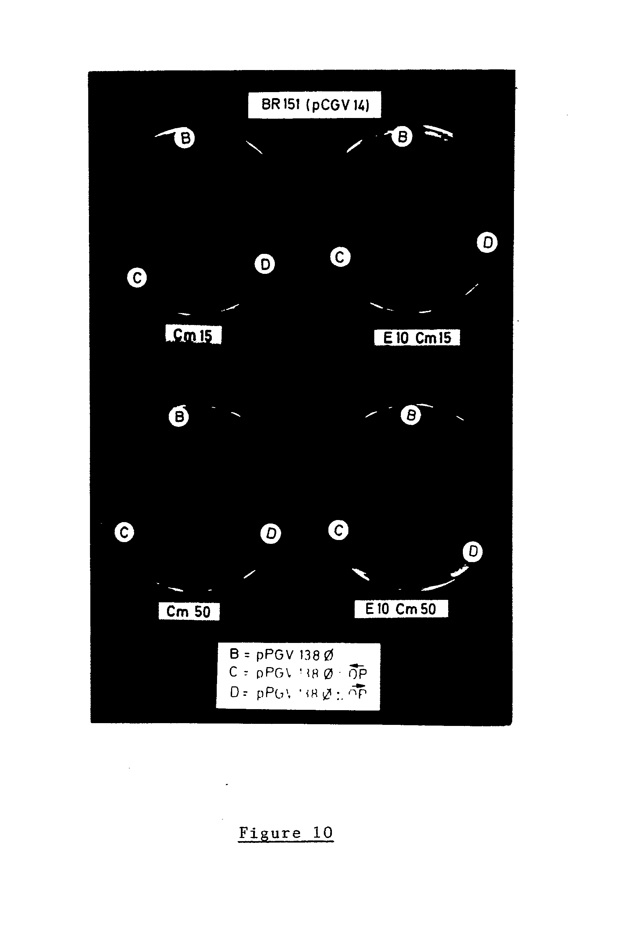

Thus, B. subtilis BR 151 strains, carrying the following plasmids, were streaked on antibiotic plates (fig. 10) :
1) pPGV138$\phi$
2) pPGV301
3) pPGV326
4) pPGV138$\phi$, pCGV14
5) pPGV301, pCGV14
6) pPGV326, pCGV14

From these growth tests the following important conclusions can be drawn :
- pPGV 301 directs a cat-86 expression level as high as pPGV138$\phi$ in the absence of repressor; thus the insertion of the operator in the correct orientation does not affect promoter activity;
- when the repressor is introduced (on the EM$^R$ plasmid pCGV14) the cM resistance of pPGV301 is considerably reduced (from over 50 $\mu$g/ml to less than 15 $\mu$g/ml) whereas that of pPGV138$\phi$ is not affected;
- the operator fragment is not functional in the wrong orientation : in this case, a lower cat-86 expression is found regardless of the presence or absence of repressor; this might be due to the opposing nature of the two promotors (i.e. the strong promotor from pPGV138$\phi$ and the weak operator-associated promotor).

The antibiotic growth tests were further substantiated and quantified by enzymatic CAT-activity assays. Table IV shows that at least a 25-fold induction can be obtained using pPGV301. This figure is almost certainly an underestimate due to the superimposed translational constraint on cat-86 gene expression. Indeed, these tests were performed using 5 $\mu$g/ml Cm for induction of mRNA translation, and this is probably not enough to fully induce the large amount of mRNA made in the absence of repressor. Further, the CAT-assays reveal that there is a certain level of repression even with the operator in the inverse orientation (compare pPGV326 with and without pCGV14).

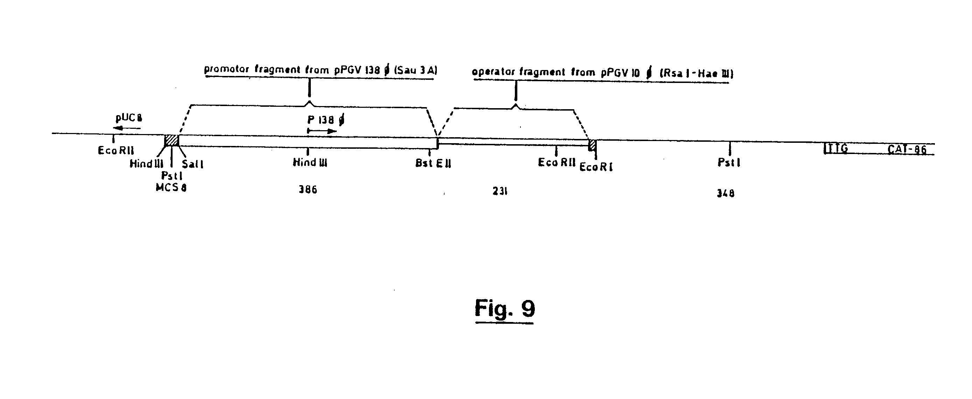

Given the above results, it can be concluded that pPGV301 is an efficient expression vector for B. subtilis. Moreover fig. 9 shows that the hybrid promotor-operator element can easily be excised from pPGV301 (e.g. using Pstl or Sall + EcoRI digestion) and inserted in another genetic background, in other words it is "portable".

## DISCUSSION AND COMPARISON WITH OTHER GENE EXPRESSION SYSTEMS

There was described a generally applicable, inducible gene expression system for Gram-positive bacteria. The crucial elements in this system, repressor and its cognate operator, are derived from a temperate bacteriophage indigenous to B. subtilis. Their interaction results in a transcriptional block of expression of a gene placed downstream of the operator. Such a negative control of transcription (or repression) has hitherto only been found in Gram-negative bacteria, but never in Gram-positives, such as B. subtilis. In the latter organisms, positive transcriptional control through the association of core RNA polymerase with new $\sigma$ factors (Losick and Pero, 1981) apparently is the only known type of gene regulation.

It is believed that this gene expression system shows some novel features, and offers advantages over similar gene expression systems, e.g. for Gram-negative (reference 25) and Gram-positive (reference 26) organisms. First of all, the operator can be isolated and fused to another, unrelated promoter, while retaining its functionality, i.e. the hybrid promoter-operator construction is still effectively controlled by the repressor. (Example : pPGV 138$\phi$). This system therefore permits a great flexibility in the choice of the promoter. As promoter, any DNA sequence functional in B. subtilis may be used, regardless of its natural origin. The promoter can exhibit any kind of transcriptional efficiency. In fact, very strong promoters (such as the veg promoter; reference 15 or the E. coli phage T5 early promotors) could be used, which would result in extremely high levels of gene expression. Such a flexibility, allowing the use of a promotor originating from the same DNA material or from DNA material of another source has not been demonstrated in the $\lambda P_L$ and $P_R$ promoter-based E. coli vectors, since in this case the operators have always been used in association with their own promoter. Yansura and Henner (1984) have recently described a regulatable expression system for B. subtilis, employing the lac operator-repressor combination from E. coli. These investigators also use a hybrid promoter-operator as transcriptional control element. An important difference, however, between their system and the one described here, concerns the manner in which the synthesis of repressor is controlled. They use a B. subtilis promoter (penP) to express the lac repressor, whereas in the present system the $\phi$105 repressor is expressed via its own promoter. This is important with respect to the problem of obtaining sufficient repression when a very strong promoter is fused to the operator.

Indeed, it has been shown that the $\phi$105 repressor stimulates its own transcription (autogenous positive regulation), much in the same way as $\lambda$ repressor does. This ensures the synthesis of high intracellular level of repressor, meeting the requirements for repression of a very strong promoter (e.g. by binding to a number of operators placed in tandem).

Therefore, in order to obtain a highly efficient, well controllable expression system for a Gram-positive host, it is advantageous to employ regulatory elements (operator-repressor) of Gram-positive origin.

A Bacillus subtilis strain comprising a first plasmid pPGV 10$\phi$ and a second plasmid pCGV28 has been deposited at the N.C.I.B. under the designation BD170 [pPGV10$\phi$, pCGV28] . (N.C.I.B. number 12014 received on 7th September 1984). This deposit has been made under the requirements of the Budapest Treaty. A second bacillus strain comprising a first plasmid pPGV301 and a second plasmid pCGV14 has also been deposited at the N.C.I.B. under the designation BR151 [pPGV301, pCGV14] (N.C.I.B. number 12170 in order to illustrate all the objects of the invention.

TABLE I.

Bacterial strains and plasmids used

| Strain or plasmid | Relevant genotype | Reference or source |
|---|---|---|
| **Bacterial strains** | | |
| **B. subtilis** | | |
| 168 | trpC2 | BGSC* |
| 168(φ105) | trpC2 lysogenic for wild-type 105 | BGSC |
| BD170 | trpC2 thr-5 | BGSC |
| BR151 | trpC2 lys-3 metB10 | Paul Lovett |
| **E. coli** | | |
| C600 | $r_K^-$ $m_K^-$ | |
| JM83 | (lac-proAB) supE thi F' lacI Z M15 proA$^+$B$^+$ | J. Messing |
| **Plasmids** | | |
| pUB110 | $Km^R$ | BGSC; Reference 24 |
| pC194 | $Cm^R$ | BGSC; Reference 16 |
| pE194 | $Em^R$ rep$^{ts}$ | BGSC; Reference 17 |
| pPL603 | $Km^R$ | Reference 1 |
| pPL608 | $Km^R$ $Cm^R$ | Reference 14 |
| pHV14/F | $Cm^R$ $Ap^R$ | Reference 19 |
| pUC4 | $Ap^R$ | Reference 2 |
| pUC8 | $Ap^R$ | Reference 2 |

\* Bacillus Genetic Stock Centre, Ohio State University.

EP 0 182 562 B1

TABLE II.

Size, CAT activity, and map position of cloned φ105 promoter fragments

| Plasmid | Insert size (bp) | CAT-specific activity [a] ($\mu$moles/h x mg protein) | Hybridization 105 _EcoRI_ fragment (E or F) |
|---|---|---|---|
| pUB110 | --- | 0.006 | |
| pPL603 | --- | 0.18 | |
| pPL608 | 300[b] | 83 | |
| pPGV2 | --- | 0.3 | |
| pPGV1 φ | 580[c] | 49 | E |
| pPGV2 φ | 250 | 13 | |
| pPGV4 φ | 650 | 129 | |
| pPGV6 φ | 350 | 48 | |
| pPGV7 φ | 800[c] | 5 | F |
| pPGV9 φ | 800[c] | 17 | F |
| pPGV10 φ | 650[c] | 13 | F |
| pPGV103 φ | 600 | N.D. | E |
| pPGV104 φ | 250 | N.D. | |
| pPGV110 φ | 450 | 104 | F |
| pPGV117 φ | 700 | 38 | F |
| pPGV120 φ | 650[c] | N.D. | E |
| pPGV123 φ | 550[c] | 38 | E |
| pPGV129 φ | 200 | 31 | |
| pPGV131 φ | 850 | N.D. | E |
| pPGV138 φ | 386[c] | 1030 | |
| pPGV139 φ | 250 | 8 | |
| pPGV201 φ | N.D. | 152 | N.D. |
| pPGV202 φ | 1000 | 143 | |

15

Table II (cont'd)

| | | | |
|---|---|---|---|
| pPGV203φ | N.D. | 36 | N.D. |
| pPGV204φ | N.D. | 71 | N.D. |
| pPGV205φ | 2000[c] | 18 | F |
| pPGV206φ | N.D. | 39 | E |
| pPGV207φ | 2250 | 262 | |
| pPGV208φ | 2100 | 3 | F |
| pPGV209φ | 1150 | N.D. | F |
| pPGV210φ | N.D. | N.D. | E |
| pPGV212φ | 3000 | N.D. | |
| pPGV214φ | N.Dv | 12 | N.D. |
| pPGV215φ | 250 | 136 | |
| pPGV216φ | N.D. | 92 | N.D. |
| pPGV217φ | N.D. | 146 | N.D. |
| pPGV220φ | 350 | 106 | |

[a] : CAT-specific activities were determined on late exponential phase B. subtilis cultures, grown in the presence of 5 μg Cm/ml (except for strains containing pUB110 and pPL603). The pellet from 1 ml culture was resuspended in 0.15 ml of 0.25 ml Tris-HCl, pH 7.8, 0.05 M EDTA, and 100 μg egg-white lysozyme was added. The suspension was incubated for 15 min at 37°C, and frozen by immersion in ethanol/dry ice. After thawing at room temperature, the lysed cells were centrifuged, and the supernatant was appropriately diluted with 0.25 M Tris·HCl, pH 7.8. Phenylmethylsulfonyl fluoride was added to a final concentration of 0.5 mM, and 5 μl of extract was incubated with 16 nmoles acetyl coenzyme A and 4 nmoles of $[^{14}C]$-chloramphenicol in 0.25 M Tris·HCl, pH 7.8 (total volume 30 μl) for 30 min at 37°C. Cm and its acetylated derivatives were extracted from the reaction mixture with 0.2 ml ethylacetate and were separated by chromatography on silica gel sheets in chloroform : methanol (95:5). The percentage acetylation of substrate was measured by excising the

radio-active spots from the chromatogram and counting by liquid scintillation spectrometry. Appropriate dilutions of extracts were used so that not more then 30% of substrate was acetylated during the assay. Protein concentration in the extracts was measured by the method of Lowry et al. (1951).

[b] : Reference 14

[c] : Insert contains at least one HindIII site. N.D., not determined.

TABLE III.

Growth of BD170 [pCGV28] , transformed with different promoter plasmids, on the indicated antibiotics (Cm, 15 $\mu$g/ml; Em, 10 $\mu$g/ml)

| Plasmid | Transformation of B. subtilis BD170 [pCGV28] at | | |
| | 33°C (Cm) | 45°C (Cm) | 37°C (Cm + Em) |
| --- | --- | --- | --- |
| pPGV2 | - | - | - |
| pPGV1φ | + | + | + |
| pPGV2φ | + | + | + |
| pPGV9φ | +/- [a] | + | - |
| pPGV10φ | +/- [a] | + | - |
| pPGV103φ | + | + | + |
| pPGV104φ | + | + | + |

Table III (cont'd)

| | | | |
|---|---|---|---|
| pPGV110 φ | + | + | + |
| pPGV123 φ | + | + | + |
| pPGV129 φ | + | + | + |
| pPGV138 φ | + | + | + |
| pPGV201 φ | + | + | + |
| pPGV202 φ | + | + | + |
| pPGV207 φ | + | + | + |
| pPGV209 φ | +/- [a] | + | - |
| pPGV211 φ | + | + | + |
| pPGV215 φ | + | + | + |
| pPGV217 φ | + | + | + |
| pPGV220 φ | + | + | + |

[a] Poor growth, as shown in Fig. 5C.

Table IV

| PLASMID (in B.s.BR151) | CAT-spec.act. ($\mu$moles/hr.mg protein) | x-fold induction |
|---|---|---|
| None | 0.21 | |
| pPGV138 φ | N.D. | |
| pPGV138 φ + pCGV14 | 280 | |
| pPGV301 | 640 | |
| pPGV301 + pCGV14 | 24.4 | 26.23 |
| pPGV326 | 195 | |
| pPGV326 + pCGV14 | 45.7 | 4.27 |

Table V Codon usage for the φ105 repressor (ORF1) and ORF2 genes

| | | ORF1 | ORF2 | | | ORF1 | ORF2 | | | ORF1 | ORF2 | | | ORF1 | ORF2 |
|---|---|---|---|---|---|---|---|---|---|---|---|---|---|---|---|
| Phe | TTT | 1 | 5 | Ser | TCT | 3 | 0 | Tyr | TAT | 2 | 8 | Cys | TGT | 0 | 0 |
| | TTC | 0 | 2 | | TCC | 1 | 2 | | TAC | 3 | 1 | | TGC | 0 | 1 |
| Leu | TTA | 3 | 4 | | TCA | 3 | 3 | Och | TAA | 0 | 0 | Umb | TGA | 0 | 0 |
| | TTG | 1 | 2 | | TCG | 1 | 0 | Amb | TAG | 1 | 1 | Trp | TGG | 0 | 2 |
| Leu | CTT | 5 | 5 | Pro | CCT | 0 | 3 | His | CAT | 0 | 4 | Arg | CGT | 4 | 1 |
| | CTC | 1 | 1 | | CCC | 1 | 1 | | CAC | 1 | 2 | | CGC | 0 | 0 |
| | CTA | 0 | 4 | | CCA | 2 | 3 | Gln | CAA | 7 | 2 | | CGA | 0 | 1 |
| | CTG | 1 | 0 | | CCG | 0 | 0 | | CAG | 2 | 2 | | CGG | 0 | 1 |
| Ile | ATT | 6 | 2 | Thr | ACT | 3 | 4 | Asn | AAT | 4 | 11 | Ser | AGT | 1 | 1 |
| | ATC | 2 | 3 | | ACC | 1 | 3 | | AAC | 1 | 3 | | AGC | 2 | 1 |
| | ATA | 4 | 5 | | ACA | 1 | 1 | Lys | AAA | 9 | 9 | Arg | AGA | 6 | 2 |
| Met | ATG | 5 | 4 | | ACG | 1 | 1 | | AAG | 5 | 4 | | AAG | 1 | 1 |
| Val | GTT | 4 | 6 | Ala | GCT | 2 | 2 | Asp | GAT | 3 | 5 | Gly | GGT | 1 | 1 |
| | GTC | 1 | 2 | | GCC | 4 | 1 | | GAC | 4 | 1 | | GGC | 3 | 0 |
| | GTA | 1 | 2 | | GCA | 4 | 7 | Glu | GAA | 14 | 4 | | GGA | 1 | 0 |
| | GTG | 1 | 2 | | GCG | 2 | 1 | | GAG | 6 | 7 | | GGG | 1 | 3 |

Total : 146 157

## Table VI. Comparison of the 105 repressor amino acid sequence with that of other repressors

| Protein | No of compared positions | Matches | % |
|---|---|---|---|
| $\lambda$ $c_I$ | 146 | 14 | 9.6 |
| $\lambda$ cro | 65 | 4 | 6.2 |
| P22 c2 | 146 | 24 | 16.4 |
| lac R | 147 | 14 | 9.5 |
| gal R | 146 | 16 | 11.0 |
| lex R | 152 | 27 | 17.8 |

LEGEND TO FIGURES

Fig 1. Structure of the promoter probe plasmid pPGV2.

A. Restriction map. The sequence originating from B. pumilis is shown as an open box with an arrow indicating the position of the cat-86 coding region (Reference 11). The enlargement shows restriction sites present in the pUC8-derived multiple cloning site (MCS8) element (Reference 2).

B. Agarose gel analysis of fragments after digestion of pPGV2 with the indicated enzymes. Lane m is a λ PstI digest of λDNA used as size markers. HindIII fragment sizes are indicated.

Fig. 2. Size determination of insert fragments in recombinant promoter plasmids.

Only a few relevant examples are shown. The complete results are summarized in Table II. E. coli 600 cells were transformed with plasmid DNA originating from individual $Cm^R$ B. subtilis transformants. Plasmid DNA purified from E. coli was digested with HindIII. Lane a, vector pPGV2 (see Fig. 1B, lane c); lanes b - c, chromosomal Sau3A fragments inserted in pPGV11c and pPGV33c; lanes d - g, phage φ105 Sau3A fragments inserted in pPGV10φ, pPGV138φ, pPGV205φ, and pPGV209φ, respectively. For lane m, see Fig. 1B.

Fig. 3. Hybridization of φ105 promoter plasmids to EcoRI-F (A) and EcoRI-E (B) fragments of φ105.

DNA (5-10 μg) of the indicated plasmids was denatured and bound to nitrocellulose, followed by hybridization to nicktranslated, $^{32}$P-labeled 105-EcoRI fragments. Phage φ105 DNA served as a positive control, whereas the vector plasmid pPGV2 and two chromosomal promoter plasmids (pPGV9c and pPGV11c) were included as negative controls. The prefix pPGV (see Tables II and III) was not used (2φ = pPGV2φ, etc.).

Individual φ105 EcoRI fragments to be used as hybridization probes were purified by agarose gel electrophoresis and subsequent electroelution. Standard conditions were used for DNA transfer to nitrocellulose, preparation of $^{32}$P-labeled DNA, and hybridization.

EP 0 182 562 B1

Fig. 4. Scheme for the construction of the repressor plasmid pCGV28.

The pE194 map is from (Reference 20). pHV14/F is described by (Reference 19). In pCGV4 and pCGV28, the heavy line represents pE194 sequences. The orientation of the EcoRI-F fragment (double line) in pCGV28 was determined by XbaI-HindIII digestion.

Fig. 5. Growth of strain BD170 [pCGV28] , transformed with (A) vector pPGV2, (B) promoter plasmid pPGV129φ, and (C) promoter plasmid pPGV10φ, on Cm plates (15 µg/ml) at 33°C and 45°C, and Em + Cm plates at 37°C.

For each promoter plasmid transformation, four independent colonies, selected on Em + Km plates at 37°C were tested.

Fig. 6. Structure of the promoter-cat-86 region in pPGV10φ.

The Sau3A promoter fragment is shown inserted in the BamHI site of pPGV2. Distances are in bp.

Fig. 7. Mapping position of the φ105 repressor gene and the early promoter Sau3A fragment.

A.  Left : gel electrophoretic pattern of fragments after digestion of φ105 fragment EcoRI-F with the indicated enzymes. Right : autoradiogram after hybridization with $^{32}$P-labeled pPGV10φ.

B.  Map of fragment EcoRI-F indicating the position of the repressor gene (c 105) and the pPGV10φ promoter $p_E$ fragment. The arrow indicates the direction of transcription from the promoter. Besides the drawn restriction sites, fragment F also contains one site for each BstEII, HpaI, and PstI.

C.  EcoRI restriction map of φ105 (Reference 21). Fragment lengths are in kb.

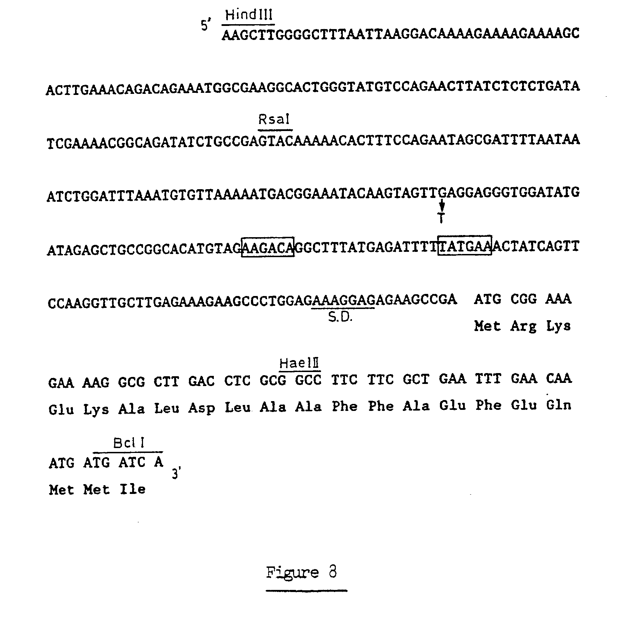

Figure 8. Nucleotide sequence of the HindIII-Sau3A(BclI) fragment containing the operator.

The RsaI and HaeIII sites delineating the operator fragment cloned in the SmaI site of pPGV138φ are indicated. Putative "-35" and "-10" hexanucleotides are boxed. A Shine-Dalgarno sequence (S.D.) is underlined, followed by the start and N-terminal amino acids of a putative coding region.

Figure 9. Structure of the hybrid promoter-operator cat-86 region in pPGV301.

Figure 10 Antibiotic resistance plate tests.

Plasmid pCGV14, containing a functional repressor, consists of the 2.3-kb PstI fragment I of φ105 inserted in pE194 cop-6 (Dhaese et al. (1985) Mol. Gen. Genet., 200, 490-492). Streaks were made from single colony transformants of each of the indicated strains. Left, without repressor; right, with repressor. C, pPGV326 (≡ pPGV138φ:: op); D, pPGV301; Cm, chloramphenicol; E, erythromycin. Concentrations are in µg/ml (≡ pTGV138φ:: op).

Figure 11 Map position and sequencing strategy of the φ105 repressor region.

The top shows a restriction map of a φ105 DNA segment around 70-75 % genome length, including EcoRI fragments H and F. The location of PstI fragment I is also indicated. The open bar shows the map position of the repressor (Cφ105). The black bar shows the position of the early promoter BclI fragment (P). The bottom shows the detailed restriction map of the repressor region. The position and direction of the two ORFs is indicated below the restriction map. Solid arrows indicate sequences derived by 5' end labelling. A broken line indicates a sequence generated by 3' end labelling. Distances are in bp. E1, EcoRI; E2, EcoRII (BstNI); E5, EcoRV; F, HinfI; H, HindIII; P, PvuII; R, RsaI; S, Sau3A. Figure 12. Nucleotide and deduced amino acid sequences of the φ105 repressor (ORF1)-ORF2 genes and their flanking regions. The sequence is shown in reversed orientation with respect to Figure 1, in order to display the sense strand. It extends from the HindIII site 272 bp upstream of the repressor-coding sequence, to an RsaI site within the φ105 EcoRI-H fragment. Nucleotide numbering starts from the GTG codon (+1). Amino acids are numbered at the right. The endpoint of the deletions in four PCGV14Δ... mutants is indicated. The promoter "-35" and "-10" regions are boxed, and the Shine-Dalgarno sequence is underlined. The dashed line in

21

ORF1 indicates a putative DNA-binding region.

Figure 13

A.  Circular map of plasmid pCGV14 (3)
The orientation of the φ105 PstI-I fragment (thin line) with respect to pE194cop-6 (thick line) was determined by digestion with XbaI + PvuII.
The position is ORF1 is indicated by the dashed arrow. Em$^R$,erythromycin resistance.
B.  Circular map of pCGV23.
This plasmid is derived from pPL703 (10) through substitution of the 850-bp HindIII-PvuII vector fragment with the 740-bp HindIII-PvuII φ105 fragment comprising ORF1. Km$^R$, kanamycin resistance; cat-86, a chloramphenicol resistance gene originating from Bacillus pumilus.

Figure 14. Hydropathy profiles of the φ105 repressor, according to the method of Hopp and Woods (31), solid line, and to that of Kyte and Doolittle (32), broken line.

Values were obtained using the average of a moving segment of 7 residues. Hydrophilic regions are found above the midline, hydrophobic are below.

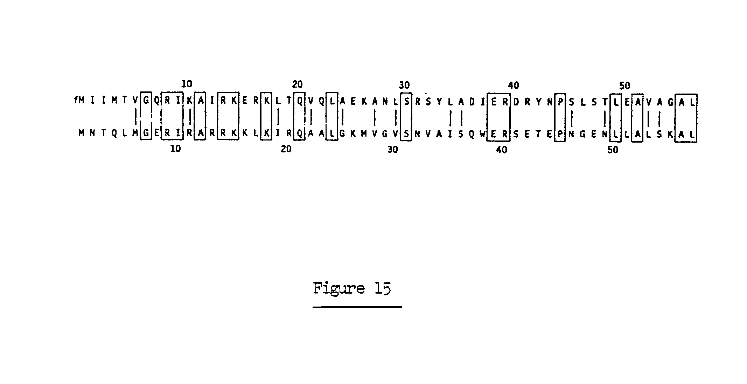

Figure 15. Alignment of the N-terminal amino acid sequences of φ105 repressor (top) and S. typhimurium phage P22 c2 repressor (bottom).

The one-letter notation for amino acids is used. Residue numbers for φ105 repressor are identical to Figure 2. In P22 c2 repressor, the N-terminal methionine is deformylated, but not removed after translation (23). Hence, numbering starts with this residue. Positions showing identical residues are boxed. Positions with a functionally rather conservative substitution are indicated by a vertical connector line.

Figure 16. Amino acid sequence homologies (one-letter notation) between several repressors in the region corresponding to the ∝2-turn-∝3 configuration of λ cI and λ cro.

Except for the φ105 repressor, data have been reproduced from (30). The amino acid positions within each individual polypeptide are shown below its sequence. The crucial positions (residues 5, 9, 15, 18) are boxed.

REFERENCES

(1) Williams, D.M., Duvall, E.J. and Lovett, P.S.: Cloning restriction fragments that promote expression of a gene in B. subtilis. J. Bacteriol. 146 (1981a) 1162-1165.
(2) Vieira, J. and Messing, J.: The pUC plasmids, an M13mp7derived system for insertion mutagenesis and sequencing with synthetic universal primers. Gene 19 (1982) 259-268.
(3) Yasbin, R.E., Wilson, G.A. and Young, F.E.: Transformation and transfection in lysogenic strain of Bacillus subtilis 168. J. Bacteriol. 113 (1973) 540-548.
(4) Birnboim, H.C. and Doly, J.: A rapid alkaline extraction procedure for screening recombinant plasmid DNA. Nucl. Acids Res. 7 (1979) 1513-1523.
(5) Goldfarb, D.S., Rodriguez, R.L. and Doi, R.H.: Translational block to expression of the E. coli Tn9-derived chloramphenicol resistance gene in Bacillus subtilis. Proc. Natl. Acad. Sci USA 79 (1982) 5886-5890.
(6) Yamamoto, K.R., Alberts, B.M., Benziger, R., Lawhorne, L. and Treiber, G.: Rapid bacteriophage sedimentation in the presence of polyethylene glycol and its application to large-scale virus purification. Virology 40 (1970) 734-744.
(7) Dubnau, D. and Davidoff-Abelson, R : Fate of transforming DNA following uptake by competent Bacillus subtilis. I. Formation and properties of the donor-recipient complex. J. Mol. Biol. 56 (1971) 209-221.
(8) Chang, S. and Cohen, S.N.: High frequency transformation of Bacillus subtilis protoplasts by plasmid DNA. Mol. Gen. Genet. 168 (1979) 111-115.
(9) Horinouchi, S. and Weisblum, B.: Nucleotide sequence and functional map of pE194, a plasmid that specifies inducible resistance to macrolide, lincosamide, and streptogramin type B antibiotics. J. Bacteriol. 150, (1982) 804-814.

(10) Rutberg, L.: Mapping of a temperate bacteriophage active on Bacillus subtilis. J. Virol. 3 (1969) 38-44.

(11) Harwood, C.R., Williams, D.M. and Lovett, P.S.: Nucleotide sequence of a Bacillus pumilis gene specifying chloramphenicol acetyl transferase. Gene 24 (1983) 163-169.

(12) Schoner, R.G., Williams, D.M. and Lovett, P.S.: Enhanced expression of mouse dihydrofolate reductase in Bacillus subtilis. Gene 22 (1983) 47-57.

(13) Duvall, E.J., Williams, D.M., Lovett, P.S., Rudolph, C., Vasantha, N. and Guyer, M.: Chloramphenicol-inducible gene expression in Bacillus subtilis. Gene 24 (1983) 171-177.

(14) Williams, D.M., Schoner, R.G., Duvall, E.J., Preis, L.H. and Lovett, P.S.: Expression of Escherichia coli trp genes and the mouse dihydrofolate reductase gene cloned in Bacillus subtilis. Gene 16 (1981b) 199-206.

(15) Moran, C.P., Lang, N., Le Grice, S.F.J., Lee, G., Stephens, M., Sonenshein, A.L., Pero, J. and Losick, R.: Nucleotide sequences that signal the initiation of transcription and translation in Bacillus subtilis. Mol. Gen. Genet. 186 (1982) 339-346.

(16) Iordanescu, S.: Three distinct plasmids originating in the same Staphylococcus aureus strain. Arch. Rom. Path. Exp. Microbiol. 35, (1976) 111-118.

(17) Weisblum, B., Graham, M.Y., Gryczan, T. and Dubnau, D.: Plasmid copy number control : isolation and characterization of high-copy number mutants of plasmid pE194. J. Bacteriol. 137 (1979) 635-643.

(18) Scheer-Abramovitz, J., Gryczan, T. and Dubnau, D.: Origin and mode of replication of plasmids pE194 and pUB110. Plasmid 6 (1981) 67-77.

(19) Uhlén, M., Flock, J.-I. and Philipson, L.: RecE-independent deletions of recombinant plasmids in Bacillus subtilis. Plasmid 5 (1981) 161-169.

(20) Gryczan, T.J., Hahn, J., Contente, S. and Dubnau, D.: Replication and incompatibility properties of plasmid pE194 in Bacillus subtilis. J. Bacteriol. 152 (1982) 722-735.

(21) Bugaichuk, U.D., Deadman, H., Ermington, J. and Savva, D. : Restriction enzyme analysis of Bacillus subtilis bacteriophage 105 DNA. J. Gen. Microbial 130 (1984) 2165-2167.

(22) Cohen, S., Chamg, A.C.Y. and Hsu, L. : Non-chromosomal antibiotic resistance in bacteria transformation of E. Coli by R. factor DNA. Proc. Natl. Acad. Sci. USA 69 (1972) 2110-2114.

(23) Flock. J.-I. : Deletion mutants of temperate Bacillus subtilis bacteriophage 105. Mol. Gen. Genet. 155 (1977) 241-247.

(24) Keggins, K.M., Lovett, P.S., and Duvall, E.J.: Molecular cloning of genetically active fragments of Bacillus DNA in Bacillus subtilis and properties of the vector plasmid pUB110. Proc. Natl. Acad. Sci. USA 75 (1978) 1423-1427.

(25) Remaut, E., Stanssens, P., and Fiers, W. (1981). Plasmid vectors for high-efficiency expression controlled by the $P_L$ promoter of coliphage lambda. Gene 15, 38-44.

(26) Yansura, D.G., and Henner, D.J. (1984). Use of the Escherichia coli lac repressor and operator to control gene expression in Bacillus subtilis. Proc. Natl. Acad. Sci. USA 81, 439-443.

## Claims

1. Process for a thermoinducible gene expression in Gram positive bacteria characterized in that this thermoinducible gene expression is realized through the insertion of two different kinds of plasmid, issued from genetic engineering and comprising respectively as concerns the first kind of plasmid the DNA material of a heterologous structural gene to be expressed preceeded by the DNA material of a promoter-operator appropriate to the Gram positive bacteria and as concerns the second kind of plasmid, the DNA material of a repressor gene whose protein product is capable of interaction with the DNA material of the promoter-operator present on the first plasmid, and the DNA material of an entity temperature sensitive for replication of the second plasmid, into the Gram positive bacteria.

2. Process according to claim 1 characterized in that the Gram positive bacteria is a Bacillus subtilis strain.

3. Process according to claims 1 to 2 characterized in that the DNA material of the promoter-operator and the DNA material of the repressor gene are issued from the same DNA material.

4. Process according to claim 3 characterized in that the DNA material from which the promoter-operator and repressor gene are issued is the genomic material of a temperate bacteriophage.

EP 0 182 562 B1

5. Process according to anyone of claims 1 to 4 characterized in that the DNA material of the entity temperature sensitive for replication of the second plasmid is a synthetic or naturally occurring DNA material.

6. Plasmid or vector composition intended to be included in Gram positive bacteria strains comprising two plasmids containing respectively
a) the DNA material of a heterologous structural gene from a procaryotic or eucaryotic cell preceeded by the DNA material of an early promoter-operator derived from a temperate phage for Bacillus subtilis,
b) the DNA material of a repressor gene, whose protein product is capable of interaction with the DNA material of the early promoter-operator present in the first plasmid, and the DNA material of an entity temperature-sensitive for replication of the second plasmid.

7. Plasmid or vector composition according to claim 6 characterized in that the DNA material of the entity temperature sensitive for replication of the second plasmid is a temperature sensitive plasmid.

8. Bacillus subtilis containing a plasmid composition comprising two plasmids containing
a) the DNA material of a heterologous structural gene from a procaryotic or eucaryotic cell preceeded by the DNA material of an early promoter-operator derived from a temperate phase for Bacillus subtilis, and
b) the DNA material of a repressor gene, whose protein product is capable of interaction with the DNA material of the early promoter-operator present in the first plasmid, and the DNA material of an entity temperature sensitive for replication of the second plasmid.

## Revendications

1. Procédé en vue de l'expression thermiquement induite d'un gène de bactérie Gram-positive, **caractérisé en ce que**
- cette expression thermiquement induite est réalisée par insertion de deux sortes différentes de plasmides produites par génie génétique et comprenant respectivement, en ce qui concerne la première sorte de plasmide le matériau d'ADN d'un gène structurel hétérologue à exprimer, précédé du matériau d'ADN d'un promoteur-opérateur approprié à la bactérie Gram-positive, et en ce qui concerne la seconde sorte de plasmide, le matériau d'ADN d'un gène répresseur dont la production protéinique est capable d'interagir avec le matériau d'ADN du promoteur-opérateur présent sur le premier plasmide, et le matériau d'ADN d'une entité sensible à la chaleur en vue de la réplication du second plasmide dans la bactérie Gram-positive.

2. Procédé selon la revendication 1, **caractérisé en ce que**
- la bactérie Gram-positive est une souche de Bacillus subtilis.

3. Procédé selon les revendications 1 ou 2, **caractérisé en ce que**
- le matériau d'ADN du promoteur-opérateur et le matériau d'ADN du gène répresseur proviennent du même matériau d'ADN.

4. Procédé selon la revendication 3, **caractérisé en ce que**
- le matériau d'ADN duquel proviennent le promoteur-opérateur et le gène répresseur est le matériau génomique d'un bactériophage tempéré.

5. Procédé selon l'une quelconque des revendications 1 à 4, **caractérisé en ce que**
- le matériau d'ADN de l'entité sensible à la chaleur en vue de la réplication du second plasmide est un matériau d'ADN de synthèse ou apparaissant naturellement.

6. Plasmide ou composé vecteur à inclure dans des souches de bactéries Gram-positives, comprenant deux plasmides qui contiennent respectivement
a) le matériau d'ADN d'un gène structurel hétérologue d'une cellule procaryotique ou eucaryotique, précédé du matériau d'ADN d'un promoteur-opérateur primitif dérivé d'un phage tempéré de Bacillus subtilis,
b) le matériau d'ADN d'un gène répresseur dont la production protéinique est capable d'interagir

24

EP 0 182 562 B1

avec le matériau d'ADN d'un promoteur-opérateur primitif présent dans le premier plasmide et avec le matériau d'ADN d'une entité sensible à la chaleur, en vue de la réplication du second plasmide.

7.  Plasmide ou composé vecteur selon la revendication 6, **caractérisé en ce que**
    - le matériau d'ADN de l'entité sensible à la chaleur en vue de la réplication du second plasmide est un plamide sensible à la chaleur.

8.  Bacillus subtilis contenant un composé plasmidique comprenant deux plasmides qui contiennent
    a) le matériau d'ADN d'un gène structurel hétérologue provenant d'une cellule procaryotique ou eucaryotique, précédé du matériau d'ADN d'un promoteur-opérateur primitif dérivé d'un phage tempéré de Bacillus subtilis, et
    b) le matériau d'ADN d'un gène répresseur, dont la production protéinique est capable d'interagir avec le matériau d'ADN du promoteur-opérateur primitif présent dans le premier plasmide et avec le matériau d'ADN d'une entité sensible à la chaleur, en vue de la réplication du second plasmide.

**Patentansprüche**

1.  Verfahren zur thermoinduzierbaren Genexpression in grampositiven Bakterien, dadurch gekennzeichnet, daß diese thermoinduzierbare Genexpression durch die Insertion von zwei verschiedenen Plasmidarten herbeigeführt wird, entstanden aus genetischer Manipulation und enthaltend, was die erste Plasmidart betrifft, das DNS-Material eines heterologen strukturellen, zur Expression zu bringenden Gens, dem das DNS-Material eines für die grampositiven Bakterien geeigneten Promoter-Operators vorangeht bzw., was die zweite Plasmidart betrifft, das DNS-Material eines Repressorgens, dessen Proteinprodukt zur Interaktion mit dem DNS-Material des auf dem ersten Plasmid vorhandenen Promoter-Operators befähigt ist, und das DNS-Material einer für Replikation des zweiten Plasmids temperaturempfindlichen Entität, in die grampositiven Bakterien.

2.  Verfahren nach Anspruch 1, dadurch gekennzeichnet, daß die grampositive Bakterie ein Bacillus-subtilis-Stamm ist.

3.  Verfahren nach Anspruch 1 oder 2, dadurch gekennzeichnet, daß das DNS-Material des Promoter-Operators und das DNS-Material des Repressorgens aus demselben DNS-Material entstanden sind.

4.  Verfahren nach Anspruch 3, dadurch gekennzeichnet, daß das DNS-Material, aus dem der Promoter-Operator und das Repressorgen entstanden sind, das genomische Material eines gemäßigten Bakteriophagen ist.

5.  Verfahren nach einem der Anspüche 1 bis 4, dadurch gekennzeichnet, daß das DNS-Material der für Replikation des zweiten Plasmids temperaturempfindlichen Entität ein synthetisches oder natürliches DNS-Material ist.

6.  Plasmid- oder Vektorzusammensetzung zur Aufnahme in grampositive Bakterien mit zwei Plasmiden, enthaltend
    a) das DNS-Material eines heterologen strukturellen Gens aus einer prokaryotischen oder eukaryotischen Zelle, dem das DNS-Material eines frühen Promotor-Operators, hergeleitet von einem gemäßigten Phagen für Bacillus subtilis, vorangeht,
    b) das DNS-Material eines Repressorgens, dessen Proteinprodukt zur Interaktion mit dem DNS-Material des in dem ersten Plasmid vorhandenen frühen Promoter-Operators befähigt ist, bzw. das DNS-Material einer für Replikation des zweiten Plasmids temperaturempfindlichen Entität.

7.  Plasmid- oder Vektorzusammensetzung nach Anspruch 6, dadurch gekennzeichnet, daß das DNS-Material der für Replikation des zweiten Plasmids temperaturempfindlichen Entität ein temperaturempfindliches Plasmid ist.

8.  Bacillus subtilis, enthaltend eine Plasmidzusammensetzung mit zwei Plasmiden, enthaltend
    a) das DNS-Material eines heterologen strukturellen Gens aus einer prokaryotischen oder eukaryotischen Zelle, dem das DNS-Material eines frühen Promoter-Operators, hergeleitet von einem gemäßigten Phagen für Bacillus subtilis, vorangeht, und

25

b) das DNS-Material eines Repressorgens, dessen Proteinprodukt zur Interaktion mit dem DNS-Material des in dem ersten Plasmid vorhandenen frühen Promoter-Operators befähigt ist, und das DNS-Material einer für Replikation des zweiten Plasmids temperaturempfindlichen Entität.

EP 0 182 562 B1

## Figure 1

A.

B.

Figure 2

Figure 3

A.

B.

Figure 4

Figure 5

| 33°C | 37°C | 45°C |
|------|------|------|
| Cm | Em+Cm | Cm |

EP 0 182 562 B1

## Figure 6

Figure 7

5′ <u>HindIII</u>
<u>AAGCTTGGGGCTTTAATTAAGGACAAAAGAAAAGAAAAGC</u>

ACTTGAAACAGACAGAAATGGCGAAGGCACTGGGTATGTCCAGAACTTATCTCTCTGATA

<u>RsaI</u>
TCGAAAACGGCAGATATCTGCCGAGTACAAAAACACTTTCCAGAATAGCGATTTTAATAA

ATCTGGATTTAAATGTGTTAAAAAATGACGGAAATACAAGTAGTTGAGGAGGGTGGATATG
                                           ↓
                                           T

ATAGAGCTGCCGGCACATGTAG│AAGACA│GGCTTTATGAGATTTT│TATGAA│ACTATCAGTT

CCAAGGTTGCTTGAGAAAGAAGCCCTGGAG<u>AAAGGAGA</u>GAAGCCGA  ATG CGG AAA
                                          S.D.            Met Arg Lys

                     <u>HaeII</u>
GAA AAG GCG CTT GAC CTC GCG GCC TTC TTC GCT GAA TTT GAA CAA
Glu Lys Ala Leu Asp Leu Ala Ala Phe Phe Ala Glu Phe Glu Gln

     <u>BclI</u>
ATG ATG ATC A  3′
Met Met Ile

Figure 8

**Fig. 9**

promotor fragment from pPGV 138 φ (Sau 3 A)    operator fragment from pPGV 10 φ (Rsa I - Hae III)

pUC 8    P 138 φ

EcoRII    Hind III    Bst E II    EcoRII EcoRI    Pst I    TTG    CAT-86

Hind III   Sall
Pst I
MCS 8    386    231    348

EP 0 182 562 B1

Figure 10

Fig. 11

5'  Hind III                    -250                                                    -200
AAGCTTTTTCCCATCCAGCATGATTATCACCTCCCGTTAAGGTATGTCTAAATTGTATGGTATTCACGACATTTT

                                      -150
GTAAAAGTCGAAATTTGACGAAATTCAAGCATTTTAAAGATTCAGAGAGTATTTATCTTGTATTTCCGTCAATTTACTAA
        Δ8(+)-100
AAAATACTTGTATTTCCGTCTTTTTTTAGTATTGTATTTCCGACATTCGGATACTATAATTGTGTCATGCCACAAGACACA
        Δ1(+)                                          +1 BclI
GTGGCACAGTGAGGCACTATGTGTTGTAAAGGAGATAG  GTG ATC ATA ATG ACT GTA GGG CAA AGA ATC
Δ15(-)    Δ82(-)                         fMet Ile Ile Met Thr Val Gly Gln Arg Ile    9
                                        ORF 1→
AAA GCC ATT AGG AAG GAA CGT AAA TTA ACC CAA GTG CAA CTG GCT GAA AAA GCC AAT CTT
Lys Ala Ile Arg Lys Glu Arg Lys Leu Thr Gln Val Gln Leu Ala Glu Lys Ala Asn Leu   29
                          100
TCA CGT TCA TAC CTT GCA GAT ATT GAA AGA GAT AGA TAC AAC CCA AGC CTT TCC ACA TTA
Ser Arg Ser Tyr Leu Ala Asp Ile Glu Arg Asp Arg Tyr Asn Pro Ser Leu Ser Thr Leu   49
                                                                    200
GAA GCA GTT GCA GGC GCG TTG GGC ATT CAG GTC TCT GCC ATT GTT GGC GAG GAA ACT CTT
Glu Ala Val Ala Gly Ala Leu Gly Ile Gln Val Ser Ala Ile Val Gly Glu Glu Thr Leu   69

ATT AAA GAA GAG CAG GCC GAA TAT AAT TCA AAA GAA GAA AAG GAC ATT GCA AAA CGT ATG
Ile Lys Glu Glu Gln Ala Glu Tyr Asn Ser Lys Glu Glu Lys Asp Ile Ala Lys Arg Met   89
                          300
GAG GAA ATA AGA AAG GAC TTA GAA AAA TCG GAC GGT CTT AGC TTT TCT GGA GAG CCC ATG
Glu Glu Ile Arg Lys Asp Leu Glu Lys Ser Asp Gly Leu Ser Phe Ser Gly Glu Pro Met  109

AGT CAA GAA GCT GTT GAG TCT CTC ATG GAA GCG ATG GAG CAC ATA GTT CGT CAA ACG CAA
Ser Gln Glu Ala Val Glu Ser Leu Met Glu Ala Met Glu His Ile Val Arg Gln Thr Gln  129
                      400
AGA ATA AAT AAA AAG TAC ACT CCA AAG AAA TAT AGA AAT GAC GAT CAA GAA TAG G  GGG
Arg Ile Asn Lys Lys Tyr Thr Pro Lys Lys Tyr Arg Asn Asp Asp Gln Glu xxx         146
                                                fMet Thr Ile Lys Asn Arg    Gly   6
                        Pvu II                    ORF 2→              500
CCT TAT ACT TTG ATA AAA GCA GCT GTG CAA AGA CTA ATT AAA AAG TAT AAA ACC AGT AAT
Pro Tyr Thr Leu Ile Lys Ala Ala Val Gln Arg Leu Ile Lys Lys Tyr Lys Thr Ser Asn   26

CCT TAT GAG CTT GCA TCA TAC ATA AAT ATA AAT GTT ATT CCA TGG AAC TTG CAT CAT GAA
Pro Tyr Glu Leu Ala Ser Tyr Ile Asn Ile Asn Val Ile Pro Trp Asn Leu His His Glu   46
                              600
ATA ATG GGT TTT TAT AAG TAT GAT AAG CGA AAT AAA TAT ATC GTT ATC AAT TCC AAC TTA
Ile Met Gly Phe Tyr Lys Tyr Asp Lys Arg Asn Lys Tyr Ile Val Ile Asn Ser Asn Leu   66

AAC CAG GCA GAA AGA ACT TTT GTG TGC TCC CAT GAA TTA GGG CAT GCA CAG TTA CAC CCA
Asn Gln Ala Glu Arg Thr Phe Val Cys Ser His Glu Leu Gly His Ala Gln Leu His Pro   86
                      700
CGG GCA AAT ACA CCA TTT ATG AAA GAG CGT ACT CTT TTC TCA GTT GAT AAA TAT GAG GTT
Arg Ala Asn Thr Pro Phe Met Lys Glu Arg Thr Leu Phe Ser Val Asp Lys Tyr Glu Val  106
                                                                    800
GAG GCA AAT ACC TTT GCG GTT GAG CTC CTT CTT CCC GAT TGG GTA GTA AGC CAA TAT AAA
Glu Ala Asn Thr Phe Ala Val Glu Leu Leu Leu Pro Asp Trp Val Val Ser Gln Tyr Lys  126
        EcoRI                                                  PstI
AAT ACT GAA TTC ACC CTT GAT GAT ATA GCT GTC ATG AAT GGG GTT CCT GCA GAG TTA GCC
Asn Thr Glu Phe Thr Leu Asp Asp Ile Ala Val Met Asn Gly Val Pro Ala Glu Leu Ala  146
                              900
CAC CTA AAA GAC CTA TCA GAG CTA AAA AAT TTT TAG CCCGAAAACAGAACATATGTTTCCAAAAAGGG
His Leu Lys Asp Leu Ser Glu Leu Lys Asn Phe xxx                                  157
                                                            1000
AGGATAGATTATCATGAACTTGATGGATGAAAACACTCCAAAGAATGTCGGGATATACGTTAGGGTTTCAACAGAAGAAC
            RsaI
AAGCAAAAGAAGGGTAC
            3'

Figure 12

Fig. 13 A

**Fig. 13 B**

Figure 14

41

Figure 15

|  | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
|  |  |  | Helix 2 | | | | | | turn | | | | Helix 3 | | | | | | | |
| λ cI | Q 33 | E | S | V | A | D | K | M | G | M | G | Q | D | G | V | G | A | L | F | M 52 |
| λ cro | Q 16 | T | K | T | A | K | D | L | G | V | Y | Q | S | A | I | N | K | A | I | H 35 |
| P22 c2 | Q 21 | A | A | L | G | K | M | V | G | V | S | N | V | A | I | S | Q | W | E | R 40 |
| Lac R | L 6 | Y | D | V | A | E | Y | A | G | V | S | Y | Q | T | V | S | R | V | V | N 25 |
| Gal R | I 4 | K | D | V | A | R | L | A | G | V | S | V | A | T | V | S | R | V | I | N 23 |
| Lex R | R 28 | A | E | I | A | S | R | L | G | F | R | S | P | N | A | A | E | E | H | L 47 |
| γδ resolvase | A 161 | S | H | I | S | K | T | M | N | I | A | R | S | T | V | Y | K | V | I | N 180 |
| φ105 R | Q 20 | V | Q | L | A | E | K | A | N | L | S | R | S | Y | L | A | D | I | E | R 39 |

Figure 16